# EUROPEAN PATENT SPECIFICATION

(11) **EP 3 677 572 B1**
(45) Date of publication and mention of the grant of the patent: **07.06.2023**
(21) Application number: 18850343.7
(22) Date of filing: 23.07.2018
(51) Int. Cl.: C07D 231/14, C07D 409/12

(54) **PRODUCTION METHOD FOR PYRAZOLE-4-CARBOXAMIDE DERIVATIVE**
VERFAHREN ZUR HERSTELLUNG EINES PYRAZOL-4-CARBOXAMIDDERIVATS
PROCÉDÉ DE PRODUCTION D'UN DÉRIVÉ DE PYRAZOLE-4-CARBOXAMIDE

(30) Priority: 28.08.2017 JP 2017163730
(43) Date of publication of application: 08.07.2020
(73) Proprietor: Japan Finechem Company, Inc., Tokyo 100-0011 (JP)
(72) Inventor: KURIHARA, Yusuke, Niigata-shi Niigata 950-3126 (JP); SATO, Koki, Niigata-shi Niigata 950-3126 (JP); WASUZU, Ayase, Niigata-shi Niigata 950-3126 (JP); YAMADA, Yu, Tokyo 100-0011 (JP)
(74) Representative: Gille Hrabal Partnerschaftsgesellschaft mbB Patentanwälte
(86) International application number: PCT/JP2018/027485
(87) International publication number: WO 2019/044266

(56) References cited:
- WO-A1-2012/055864
- WO-A1-2015/118793
- WO-A1-2016/016298
- WO-A1-2017/223086
- JP-A- 2009 509 935
- JP-A- 2013 542 213
- JP-A- 2014 523 425
- JOSHUA BRITTON ET AL: "Synthesis of Celecoxib, Mavacoxib, SC-560, Fluxapyroxad, and Bixafen Enabled by Continuous Flow Reaction Modules : Synthesis of Celecoxib, Mavacoxib, SC-560, Fluxapyroxad, and Bixafen Enabled by Continuous Flow Reaction Modules", EUROPEAN JOURNAL OF ORGANIC CHEMISTRY, vol. 2017, no. 44, 23 October 2017 (2017-10-23), pages 6566-6574, XP055458473, DE ISSN: 1434-193X, DOI: 10.1002/ejoc.201700992

## Description

### Technical Field

The present invention relates to a method of producing a pyrazole-4-carboxamide derivative.

### Background Art

Pyrazole-4-carboxamide derivatives are useful as agricultural chemicals or intermediates thereof, and so on. For example, PTL 1 (WO 2013/186325 A) describes fluindapyr; PTL 2 (WO 2003/070705 A) describes bixafen; PTL 3 (WO 2006/087343 A) describes fluxapyroxad; PTL 4 (WO 2007/115765 A) describes isopyrazam; PTL 5 (WO 2007/048556 A) describes benzovindiflupyr; and PTL 6 (EP 0737682 A) describes penthiopyrad. These have excellent effects as an agricultural fungicide and are widely used in the world for controlling crop diseases.

As a method which is known as the production method of a carboxamide derivative, a method of allowing an active type of the corresponding carboxylic acid, for example, an acid chloride, to react with an amine to obtain the carboxamide derivative, is described in PTL 2 (WO 2003/070705 A), PTL 7 (WO 2007/009717 A), and PTL 8 (WO 2007/031323 A).

In addition, a method of allowing a carboxylic acid and an amine to react with each other in the presence of a condensing agent, such as N,N'-dicyclohexylcarbodiimide and bis(2-oxo-3-oxazolidinyl)phosphoryl chloride, to obtain a carboxamide is described in PTL 3 (WO 2006/087343 A) and PTL 9 (WO 2005/123690 A).

In the aforementioned method, it is needed that after converting the corresponding carboxylic acid ester into a carboxylic acid through hydrolysis, the acid chloride is prepared by using a chlorinating agent, such as thionyl chloride and oxalyl chloride, and there were involved such problems that the number of steps to reach the carboxamide is long, that a reagent which is dangerous in handling for preparation of the acid chloride must be used, and that large quantities of waste acid and waste water come out after the reaction.

In addition, in the method of using a condensing agent, in addition to the fact that a hydrolysis step of the carboxylic acid ester is needed similar to the above, there were involved such problems that wastes which are difficultly separated from the target material are generated after the reaction, and that the bis(2-oxazolidinyl)phosphoryl chloride has carcinogenicity and is dangerous in handling.

Meanwhile, in an aminolysis reaction capable of obtaining a target carboxamide by allowing a carboxylic acid ester and an amine to react directly with each other, the production process is shortened, and since the acid chlorination is not adopted, it is not needed to use a dangerous reagent, and safety in the operation becomes high. In addition to the above, it becomes possible to reduce wastes, such as waste acid and waste water. Thus, such a method may become an industrially advantageous production method.

Examples of the background art of the production method of a pyrazole-4-carboxamide derivative using the aminolysis reaction in the presence of a base include the following patent literatures: PTL 10 (WO 2012/055864 A) and PTL 11 (WO 2012/175511 A).

PTL 10 (WO 2012/055864 A) describes that the pyrazole-4-carboxamide derivative can be produced from a pyrazole-4-carboxylic acid ester and an amine by using a bulky non-nucleophilic base, such as potassium tert-butoxide. However, the base is limited to the non-nucleophilic base, and silica gel chromatography is needed for purification of the target material, and therefore, it is hard to say that the foregoing method is an industrial production method.

In addition, PTL 11 (WO 2012/175511 A) describes that the pyrazole-4-carboxamide derivative can be produced from a pyrazole-4-carboxylic acid ester and an amine by using a metal alkoxide as the base. However, for completion of the reaction, a step of reducing the pressure in the system and removing the by-produced alcohol through azeotropic distillation with toluene as a solvent is needed, and from the standpoint of complicated operation and increase of waste liquids, it is hard to say that the method is advantageous as an industrial production method.

### Citation List

### Patent Literature

PTL 1: WO 2013/186325 A
PTL 2: WO 2003/070705 A
PTL 3: WO 2006/087343 A
PTL 4: WO 2007/115765 A
PTL 5: WO 2007/048556 A
PTL 6: EP 0737682 A
PTL 7: WO 2007/009717 A
PTL 8: WO 2007/031323 A
PTL 9: WO 2005/123690 A
PTL 10: WO 2012/055864 A
PTL 11: WO 2012/175511 A

### Summary of Invention

### Technical Problem

The present invention is one solving the aforementioned problems involved in the background art and is to provide an industrial production method in which a pyrazole-4-carboxamide derivative that is useful as an agricultural fungicide can be obtained simply and in high yield and high purity as compared with the conventional method.

### Solution to Problem

The present inventors made extensive and intensive investigations regarding a method of producing a pyrazole-4-carboxamide derivative of the formula (1) through an aminolysis reaction of a pyrazole carboxylic acid ester of the formula (2) and an amine of the formula (3) in a solvent in the presence of a base according to the following reaction formula (A). As a result, surprisingly, it has been found that the reaction can be completed without removing a by-produced alcohol or phenyl, which is needed to be removed outside the system in the general aminolysis reaction, outside the system, thereby leading to accomplishment of the present invention. Specifically, the present invention is to provide an industrial production method in which not only the pyrazole-4-carboxamide derivative represented by the formula (1) is obtained by a simple operation and in high yield and high purity, but also it is possible to reduce the quantities of waste acid and waste water.

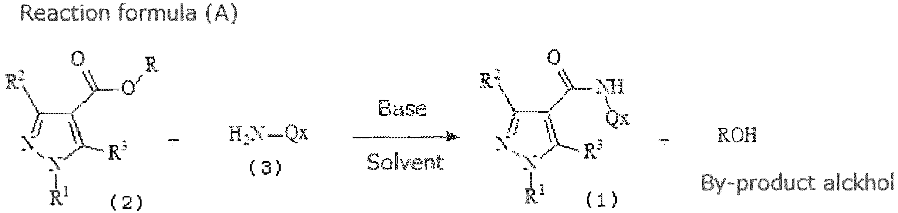

R, R¹, R², R³, and Qx in the formulae (1), (2), and (3) are respectively described later.

### Advantageous Effects of Invention

In accordance with the production method of a pyrazole-4-carboxamide derivative according to the present invention, it is possible to obtain the pyrazole-4-carboxamide derivative which is useful as an agricultural fungicide, simply and in high yield and high purity as compared with the conventional method by a short process and a simple operation, and the production method of the present invention can be adopted as an industrial production method.

### Description of Invention

The invention is defined in the appended claims. Embodiments not encompassed by the claims are provided for reference purposes.

The method of producing a pyrazole-4-carboxamide derivative according to the present invention is hereunder specifically described on a basis of the reaction formula (A).

The present invention relates to a production method of a pyrazole-4-carboxamide derivative represented by the formula (1) through an aminolysis reaction of a pyrazole-4-carboxylic acid ester of the formula (2) and an amine of the formula (3) in a solvent in the presence of a base according to the reaction formula (A) and is an invention of the production method in which on the occasion of reaction, the reaction can be completed without removing a by-produced alcohol or phenol outside the reaction system.

The pyrazole-4-carboxamide derivative obtained by the aforementioned reaction is a compound represented by the formula (1). In addition, the pyrazole-4-carboxamide derivative represented by the formula (1) includes not only a single optical isomer and a diastereoisomer but also morphologies of a racemic mixture, a diastereoisomer mixture, and a partially separated mixture of such a compound, caused by the structure of the amine represented by the formula (2).

Here, the racemic mixture and the diastereoisomer mixture each mean a mixture of isomers thereof (racemate and diastereomer); and the partially separated mixture means a mixture remained resulting from separation of a part of isomers from isomers constituting a racemic mixture and a diastereoisomer mixture (for example, in the case of a racemate, a mixture of isomers in which as a result of separation of a part of the R-form or the S-form, a ratio of the R-form and S-form has changed from a typical value of 1/1 to a value of not 1/1).

The compound represented by the formula (1) is one mentioned below.

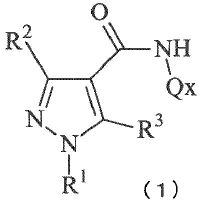

In the formula (1), R¹ is a hydrogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, preferably a C1-C4 alkyl group, and especially preferably a methyl group.

R² is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, preferably a C1-C4 haloalkyl group, and especially preferably a difluoromethyl group or a trifluoromethyl group.

R³ is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, and especially preferably a hydrogen atom.

Qx that is an amine residue is one mentioned later.

The pyrazole-4-carboxylic acid ester represented by the formula (2), which is one of the starting substances, is one mentioned below.

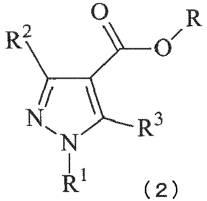

In the formula (2), R¹ is a hydrogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, preferably a C1-C4 alkyl group, and especially preferably a methyl group.

R² is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, preferably a C1-C4 haloalkyl group, and especially preferably a difluoromethyl group or a trifluoromethyl group.

R³ is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, and especially preferably a hydrogen atom.

R is a C1-C4 alkyl group or an optionally substituted phenyl group, preferably a C1-C4 alkyl group, and especially preferably an ethyl group.

The amine represented by the formula (3), which is to be allowed to react with the pyrazole-4-carboxylic acid ester represented by the formula (2) is one mentioned below.

H₂N-Qx (3)

In the formula (3), Qx represents any substituent (amine residue) of Q1, Q2, Q3, Q4, Q5, and Q6.

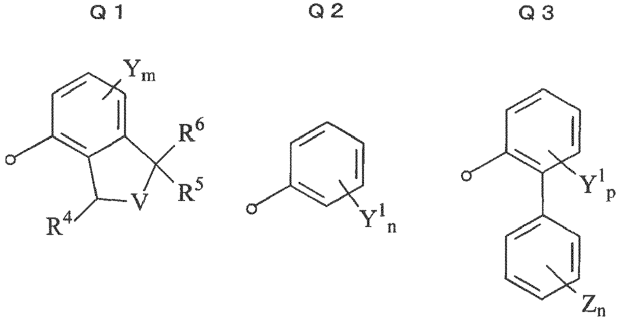

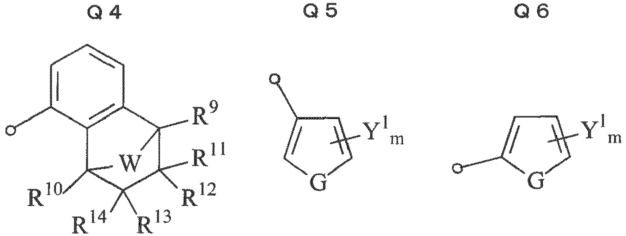

In Q1, R⁴, R⁵, and R⁶ are the same as or different from each other and are each a C1-C4 alkyl group, a C1-C4 haloalkyl group, a C3-C6 cycloalkyl group, or a C3-C6 halocycloalkyl group, provided that R⁵ and R⁶ may be bonded to each other to form a C3-C6 cycloalkyl group; and preferably a C1-C4 alkyl group. In particular, it is preferred that R⁴, R⁵, and R⁶ are each a methyl group.

V represents CH(R⁷), N(R⁸), an oxygen atom, or a sulfur atom; and R⁷ and R⁸ are each a hydrogen atom, a C1-C4 alkyl group, a C1-C4 haloalkyl group, a C3-C6 cycloalkyl group, or a C3-C6 halocycloalkyl group. In particular, it is preferred that in CH(R⁷), R⁷ is a hydrogen atom.

Y represents a halogen atom, a C1-C4 alkyl group, a C1-C4 haloalkyl group, a C1-C4 alkoxy group, a C1-C4 haloalkoxy group, an SH group, a C1-C4 alkylthio group, or a C1-C4 haloalkylthio group, preferably a halogen atom, and especially preferably a fluorine atom.

m is an integer of 0 to 3, preferably 0 or 1, and especially preferably 1. In addition, the substitution position of the fluorine atom is especially preferably the 7-position of the indane-amine.

In Q2, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and preferably a hydrogen atom, a C1-C6 alkyl group, or a halogen atom.

n represents an integer of 1 to 5, and when n is 2, 3, 4, or 5, then Y¹'s may be the same as or different from each other.

In Q3, Y¹ and Z each represent a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and preferably a hydrogen atom or a halogen atom.

p represents an integer of 1 to 4, and when p is 2, 3, or 4, then Y¹'s may be the same as or different from each other.

n represents an integer of 1 to 5, and when n is 2, 3, 4, or 5, then Z's may be the same as or different from each other.

In Q4, R⁹, R¹⁰, R¹¹, R¹², R¹³, and R¹⁴ are the same as or different from each other and each represent a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and preferably a hydrogen atom or a C1-C6 alkyl group. In addition, R¹¹ and R¹² may be bonded to each other to form a C3-C6 cycloalkyl group and R¹³ and R¹⁴ may be bonded to each other to form a C3-C6 cycloalkyl group.

W represents a methylene group, a methine group substituted with a C1-C6 haloalkyl group, or a terminal end-substituted vinyl group represented by the formula (4).

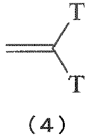

In the formula, T represents a C1-C6 haloalkyl group or a halogen atom, and preferably a chlorine atom.

In Q5, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and preferably a C1-C6 alkyl group.

m represents an integer of 1 to 3, and when m is 2 or 3, then Y¹'s may be the same as or different from each other.

G represents an oxygen atom, a sulfur atom, or N(R¹⁵), and R¹⁵ represents a hydrogen atom or a C1-C6 alkyl group, and G is preferably a sulfur atom.

In Q6, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and preferably a C1-C6 alkyl group.

m represents an integer of 1 to 3, and when m is 2 or 3, then Y¹'s may be the same as or different from each other.

G represents an oxygen atom, a sulfur atom, or N(R¹⁵), and R¹⁵ represents a hydrogen atom or a C1-C6 alkyl group, and G is preferably a sulfur atom.

Examples of the pyrazole-4-carboxamide derivative represented by the formula (1) include:
3-difluoromethyl-N-(7-fluoro-1,1,3-trimethyl-4-indanyl)-1-methyl-4-pyrazole carboxamide: a general name: fluindapyr,
3-(difluoromethyl)-1-methyl-N-(3',4'-dichloro-5-fluorobiphenyl-2-yl)-pyrazole-4-carboxamide: a general name: bixafen,
3-(difluoromethyl)-1-methyl-N-(3',4',5'-trifluorobipenyl)-pyrazole-4-carboxamide: a general name: fluxapyroxad,
a mixture of a syn-form: 3-(difluoromethyl)-1-methyl-N-((1RS,4SR,9RS)-1,2,3,4-tetrahydro-9-isopropyl-1,4-methanonaphthalen-5-yl)pyrazole-4-carboxamide and an anti-form: 3-(difluoromethyl)-1-methyl-N-((1RS,4SR,9SR)-1,2,3,4-tetrahydro-9-isopropyl-1,4-methanonaphthalen-5-yl)pyrazole-4-carboxamide: a general name: isopyrazam,
3-(difluoromethyl)-1-methyl-N-(9-(dichloromethylene)-1,2,3,4-tetrahydro-1,4-methanonaphthalen-5-yl)pyrazole-4-carboxamide: a general name: benzovindiflupyr, and
(RS)-N-[2-(1,3-dimethylbutyl)-3-thienyl]-1-methyl-3-(trifluoromethyl)-1H-pyrazole-4-carboxamide: a general name: penthiopyrad.

Examples of the pyrazole-4-carboxylic acid ester represented by the formula (2) and the amine represented by the formula (3), all of which are used for the production, include compounds having a substituent corresponding to the pyrazole-4-carboxamide derivative represented by the formula (1), respectively.

The production method of the pyrazole-4-carboxamide derivative of the present invention is a method of producing the pyrazole-4-carboxamide derivative represented by the formula (1) through an aminolysis reaction of the pyrazole-4-carboxylic acid ester represented by the formula (2) and the amine represented by the formula (3) in a solvent in the presence of a base and is characterized such that the reaction is completed without removing the by-produced alcohol or phenol outside the system.

In the present invention, in the aminolysis reaction, as for a charging ratio of the pyrazole-4-carboxylic acid ester represented by the formula (2) and the amine represented by the formula (3), the amount of the pyrazole-4-carboxylic acid ester is in a range of 1.0 to 2.0 equivalents, and preferably in a range of 1.0 to 1.5 equivalents to 1.0 equivalent of the amine.

In the present invention, in the aminolysis reaction, the base to be used is preferably a metal alkoxide, and more preferably a non-bulky metal alkoxide. Specifically, examples thereof include lithium methoxide, lithium ethoxide, sodium methoxide, sodium ethoxide, potassium methoxide, and potassium ethoxide, with sodium methoxide and sodium ethoxide being especially preferred.

The use amount of the base is preferably in a range of 1.0 to 7.0 equivalents, more preferably in a range of 1.0 to 5.0 equivalents, still more preferably in a range of 1.0 to 3.0 equivalents, especially preferably in a range of 1.0 to 2.5 equivalents, and most preferably in a range of 1.4 to 2.5 equivalents to 1.0 equivalent of the amine represented by the formula (3). When the use amount of the base falls within this range, the target material can be obtained in a high yield without removing the by-produced alcohol or phenol outside the system, and hence, such is preferred. In the case where the use amount of the base is less than 1.0 equivalent, there is a tendency that the reaction does not proceed, and the yield does not increase.

In the present invention, the solvent which is used in the aminolysis reaction is preferably an aprotic solvent. Specific examples of the solvent include amide-based solvents, such as N,N-dimethylacetamide, N-methylpyrrolidone, N-ethylpyrrolidone, and N,N-dimethylformamide; sulfur-containing solvents, such as dimethyl sulfoxide; hydrocarbon-based solvents, such as benzene, toluene, and xylene; and halogen-based solvents, such as dichloromethane and dichloroethane. In addition, of these aprotic solvents, N,N-dimethylacetamide, N-methylpyrrolidone, N-ethylpyrrolidone, N,N-dimethylformamide, and dimethyl sulfoxide are preferred, with N,N-dimethylacetamide, N-methylpyrrolidone, N,N-dimethylformamide, and dimethyl sulfoxide being especially preferred.

The solvent which is used herein refers to a specified aprotic solvent that is the reaction solvent to be used on the occasion of performing the aminolysis reaction. However, in the case of using this aprotic solvent, even when a solvent existent in the reaction system is a mixed solvent containing other solvent than this aprotic solvent, there is no objection so far as the effects of the present invention are brought. The aprotic solvent to be used herein for the reaction becomes the solvent to be used on the occasion of the reaction as referred to in the present invention. In this case, examples of the case where other solvent is existent in such a reaction system include a case where a small amount of the solvent to be caused due to the raw material used is incorporated into the reaction system.

As the solvent which is incorporated in a small amount, for example, in the case where a source material to be used as the raw material is diluted with a solvent different from the solvent on the occasion of performing the aminolysis reaction, when using this raw material, the solvent which dissolves the raw material therein, for example, toluene, or the solvent which dissolves the base to be used as a catalyst (for example, an alcohol in the metal alkoxide), is occasionally partially incorporated into the reaction solvent.

The water content in such a reaction solvent is preferably 0.5% by mass or less, and especially preferably 0.1% by mass or less. In addition, these aprotic solvents may be used alone; or two or more thereof may be mixed and used as a mixed solvent, and as for a mixing ratio thereof, any arbitrary proportion is adaptable.

The use amount of the solvent is in a range of 0.5 to 10.0 equivalents, and preferably in a range of 2.0 to 5.0 equivalents relative to the amine represented by the formula (3), and the concentration of the amine is preferably 50% by mass or less. In the case of performing the reaction by using the solvent under such a condition, the target material can be obtained in high purity and high yield.

In the present invention, in the aminolysis reaction, the reaction temperature is generally in a range of -20°C to 140°C, and preferably in a range of 20°C to 90°C.

In the present invention, in the aminolysis reaction, the reaction time is generally in a range of 1 to 10 hours, and preferably in a range of 2 to 5 hours.

In the present invention, in the case where a metal alkoxide is used as the base in an excessive amount as 1.0 to 7.0 equivalents relative to the aforementioned amine, and preferably, the reaction is performed in the aforementioned aprotic solvent, the reaction can be completed even when the alcohol or phenol generated owing to the aminolysis reaction between the ester and the amine is not removed from the reaction system. Although, the reason for this is not elucidated yet, it may be estimated that in the case where the carboxamide as a target material, which is formed along the progress of reaction, takes in a metal ion (e.g., a sodium ion) in the reaction system to form a metal salt, the reaction equilibrium shifts to the direction of carboxamide formation, whereby the reaction is possibly completed without causing a reverse reaction. In addition, in this case, it may also be considered to be one of causes that taking-in of a metal ion by the carboxamide is promoted by an interaction with the solvent to be used for the reaction.

As a result, by adding water to the resulting reaction mixture and performing cooling, the resulting pyrazole-4-carboxamide derivative is deposited in the organic layer, whereby the target material can be given in high yield and high purity.

The amount of water needed for deposition is about 1.0 to 7.0 in terms of a mass ratio relative to the used solvent, and it is about 10.0 to 180.0 in terms of a mass ratio on a basis of the amine.

In the present invention, the pyrazole-4-carboxamide derivative can be given through a simple operation of adding water after completion of the reaction as mentioned above. However, prior to this, it is preferred that before depositing the pyrazole-4-carboxamide derivative by the addition of water to the reaction mixture after the end of the reaction, water is added to the reaction mixture after the end of the reaction to perform washing, and the aqueous layer is separated to remove an unnecessary material from the organic layer containing the target material. The amount of water when performing washing is in a range of 5 to 150 equivalents, and preferably in a range of 10 to 50 equivalents on a basis of the amine represented by the formula (3). The washing can be, for example, performed in the following manner: water is added to the reaction mixture, stirring is performed, and the resultant is allowed to stand, thereby separating the organic layer and the aqueous layer from each other, followed by removing the aqueous layer.

On the occasion of separating the organic layer and the aqueous layer from each other, in order to enhance the separation state, an alkali metal hydroxide, such as potassium hydroxide and sodium hydroxide, or an alkaline earth metal hydroxide, such as calcium hydroxide can also be added. In addition, by adding an alkali metal chloride, such as potassium chloride and sodium chloride, or an alkaline earth metal chloride, such as calcium chloride, the separation between the organic layer and the aqueous layer can also be made easy owing to a salting effect or the like.

In summary, in the present invention, after completion of the reaction, by adding water, the target material can be deposited and obtained; however, adoption of a method in which the addition of water is performed in two stages such that water is first added to perform washing and separate the organic layer and the aqueous layer from each other, and water is again added to the separated organic layer to deposit the target material is preferred from the standpoint that an inorganic material (mainly, the alkali metal hydroxide and the like) in the reaction mixture can be removed by water washing and that the purity of the pyrazole-4-carboxamide derivative can be increased.

However, even in the case where the liquid separation between the organic layer and the aqueous layer as mentioned above is not performed, when a crystal of the pyrazole-4-caroxamide derivative which has been deposited by the addition of water is rinsed with water, the purity equal to that in the case of performing the liquid separation can be achieved. In this case, the amount of water for rinsing is required to be about 2 to 10 times that in the case of liquid separation, so that the use amount of water somewhat increases; however, the purity and yield of the resulting pyrazole-4-carboxamide derivative do not change.

As mentioned above, the isolation of the target pyrazole-4-carboxamide derivative can be achieved by adding water directly to the reaction mixture after the end of the reaction; or adding water to the organic layer which has been obtained by adding water to the reaction mixture and performing washing and separation, to deposit the pyrazole-4-carboxamine derivative as a crystal, followed by performing solid-liquid separation. In addition, though the temperature on the occasion of adding water to the reaction mixture after the end of the reaction varies with the reaction temperature, the solubility of the resulting pyrazole-4-carboxamide derivative, and the amount of the organic layer, it may be a temperature at which the target material is not decomposed. In general, the temperature is preferably in a range of about 20°C to 100°C, and more preferably in a range of about 40°C to 90°C.

Subsequently, by performing cooling, the crystal can be obtained. On this occasion, regardless of whether or not performing the washing and separation, in all of the methods, it is preferred that the separated crystal is rinsed, thereby washing the mother liquid attached to the crystal.

By drying the resulting crystal, the pyrazole-4-carboxamide derivative represented by the formula (1) in a high purity can be obtained. On this occasion, a purification process, such as distillation, recrystallization, and column chromatography, is not required at all.

### Examples

The present invention is hereunder described in detail on a basis of Examples, but it should be construed that the present invention is not limited by these Examples.

The pyrazole-4-carboxamide derivatives represented by the formula (1), which were prepared according to the present invention, are shown in Table 1.

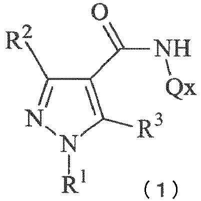

In the formula, R¹, R², R³, and Qx are the same as the contents mentioned previously.

**Table 1**

| Compound No. | R¹ | R² | R³ | Qx | Compound No, | R¹ | R² | R³ | Qx |
|---|---|---|---|---|---|---|---|---|---|
| 1 | CH₃ | CF₂H | H | Q1 | 2 | CH₃ | CF₂H | H | Q2 |
| | | | | 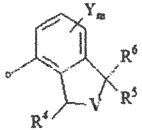 | | | | | 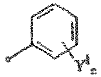 |
| | | | | | | | | | Y¹n |
| | | | | | | | | | H |
| | | | | V CH₂ | 3 | | | | 3-CH₃ |
| | | | | | 4 | | | | 3-F |
| | | | | R⁴,R⁵,R⁶ CH₃ | 5 | | | | 2,6-(CH₃)₂ |
| | | | | | 9 | CH₃ | CF₂H | H | Q4 |
| | | | | Ym | | | | | 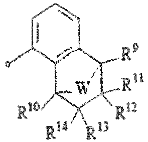 |
| | | | | 7-F | | | | | |
| 6 | CH₃ | CF₂H | H | Q3 | | | | | |
| | | | | 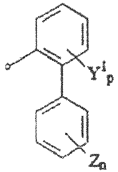 | | | | | |
| | | | | | | | | | R⁹∼R¹⁴ |
| | | | | | | | | | H |
| | | | | | | | | | W |
| | | | | | | | | | 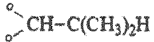 |
| | | | | Y¹ₚ | 10 | | | | 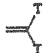 |
| | | | | H | | | | | |
| | | | | Zₙ | | | | | T |
| | | | | H | | | | | Cl |
| 7 | | | | Y¹ₚ | 11 | CH₃ | CF₃ | H | Q5 |
| | | | | 4-F | | | | | 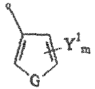 |
| | | | | Zₙ | | | | | |
| | | | | 3',4'-(Cl)₂ | | | | | |
| 8 | | | | Y¹ₚ | | | | | G |
| | | | | H | | | | | S |
| | | | | Zₙ | | | | | 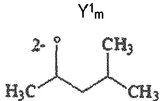 |
| | | | | 3',4',5'-(F)₃ | | | | | |

The production of the 4-pyrazolecarboxamides enumerated in Table 1 is hereunder described by reference to the Examples of the present invention, but it should be construed that the present invention is not limited thereto.

The structure of the target material was confirmed by means of the ¹H-NMR spectrum measurement at 500 MHz, and an area percentage measured by HPLC was used as the purity.

Ethyl 3-(difluoromethyl)-1-methyl-1H-pyrazole-4-carboxylate and ethyl 3-(trifluoromethyl)-1-methyl-1H-pyrazole-4-carboxylate used in the Examples were prepared by the method described in WO 2006/090778 A.

With respect to the amine, those which are commercially available were purchased and used, whereas those which are not commercially available were prepared following the reference literatures and used. The reference literatures regarding the amine preparation method are described in the following Examples.

The structure of the target material was confirmed by means of the ¹H-NMR spectrum measurement at 500 MHz, and the purity was determined by preparing a calibration curve using a standard material by HPLC and adopting the absolute calibration curve method.

### Example 1

Synthesis of 3-difluoromethyl- N- (7 -fluoro -1,1,3-trimethyl-4-indanyl)-1-methyl-4-pyrazole carboxamide (Compound No. 1: a general name: fluindapyr) Synthesis Example 1-1

To 33.9 mL of N-methylpyrrolidone in a 200-mL four-necked flask, 79.8 g (82.6 mmol) of a 21.1% by mass toluene solution of ethyl 3-(difluoromethyl)-1-methyl-1H-pyrazole-4-carboxylate and 64.9 g (70.4 mmol) of a 21.0% by mass toluene solution of 7-fluoro-1,1,3-trimethyl-4-aminoindane were added.

The 7-fluoro-1,1,3-trimethyl-4-aminoindane to be used for the reaction was prepared by reference to EP 0654464 A or the like.

Subsequently, the contents were subjected to vacuum concentration to recover 105.8 g of toluene, thereby obtaining a N-methylpyrrolidone solution of ethyl 3-(difluoromethyl)-1-methyl-1H-pyrazole-4-carboxylate and 7-fluoro-1,1,3-trimethyl-4-aminoindane in which 6.8% mass of toluene remained. Subsequently, 10.49 g (154.2 mmol) of sodium ethoxide was added, and the contents were stirred at an internal temperature of 83.6°C for 1.5 hours. The reaction mixture was cooled to 70°C, and then, 34.3 g of water was added to wash the organic layer. After separating the aqueous layer, 104.61 g of water was added to the organic layer, and cooling to 10°C was performed, whereby the target 3-difluoromethyl-N-(7-fluoro-1,1,3-trimethyl-4-indanyl)-1-methyl-4-pyrazole carboxamide was deposited as a crystal. The deposited crystal was separated by means of filtration and washed with 12.3 g of water.

The crystal after washing was dried. As a result, 24.4 g (66.7 mmol) of 3-difluoromethyl-N-(7-fluoro-1,1,3-trimethyl-4-indanyl)-1-methyl-4-pyrazole carboxamide was obtained as a white crystal. The yield was 94.5%, and the purity was 96.0%, and a purification operation, such as recrystallization, was not needed. Melting point: 170.8°C
¹H-NMR (1H resonance frequency: 500 MHz, measurement solvent: CDCl₃, internal standard substance: tetramethylsilane)

δ value (ppm): 1.43 (3H,d), 1.38 (3H,s), 1.44 (3H,s), 1.66 (1H,dd), 2.21 (1H,dd), 3.38 (1H,m), 3.93 (3H,s), 6.81 (1s,bs), 6.95 (1H,t), 6.70 (1H,m), 7.81 (1H,bs), 8.03 (1H,bs)

### Synthesis Example 1-2

To 8.65 mL of N-methylpyrrolidone in a 100-mL four-necked flask, 31.84 g (32.9 mmol) of a 21.1% by mass toluene solution of ethyl 3-(difluoromethyl)-1-methyl-1H-pyrazole-4-carboxylate and 51.74 g (30.0 mmol) of a 11.2% by mass toluene solution of 7-fluoro-1,1,3-trimethyl-4-aminoindane were added.

The contents were subjected to vacuum concentration to recover 59.4 g of toluene, thereby obtaining a N-methylpyrrolidone solution of ethyl 3-(difluoromethyl)-1-methyl-1H-pyrazole-4-carboxylate and 7-fluoro-1,1,3-trimethyl-4-aminoindane in which 13.3% mass of toluene remained. Subsequently, 2.97 g (43.6 mmol) of sodium ethoxide was added, and the contents were stirred at an internal temperature of 75.2°C for 6.0 hours. The reaction mixture was cooled to 73.0°C, and 19.9 g of water was added to the organic layer, and cooling to 0°C was performed, whereby the target 3-difluoromethyl-N-(7-fluoro-1,1,3-trimethyl-4-indanyl)-1-methyl-4-pyrazole carboxamide was deposited as a crystal. The deposited crystal was separated by means of filtration and washed with 50.0 g of water.

The crystal after washing was dried. As a result, 9.64 g (26.4 mmol) of 3-difluoromethyl-N-(7-fluoro-1,1,3-trimethyl-4-indanyl)-1-methyl-4-pyrazole carboxamide was obtained as a white crystal. The yield was 88.1%, and the purity was 96.3%, and a purification operation, such as recrystallization, was not needed. Melting point: 170.8°C
¹H-NMR (1H resonance frequency: 500 MHz, measurement solvent: CDCl₃, internal standard substance: tetramethylsilane)

δ value (ppm): 1.43 (3H,d), 1.38 (3H,s), 1.44 (3H,s), 1.66 (1H,dd), 2.21 (1H,dd), 3.38 (1H,m), 3.93 (3H,s), 6.81 (1s,bs), 6.95 (1H,t), 6.70 (1H,m), 7.81 (1H,bs), 8.03 (1H,bs)

### Synthesis Example 1-3

First of all, from the toluene solution of ethyl 3-(difluoromethyl)-1-methyl-1H-pyrazole-4-carboxylate which was used as the starting raw material in Synthesis Example 1-1, the toluene was removed with an evaporator, to obtain ethyl 3-(difluoromethyl)-1-methyl-1H-pyrazole-4-carboxylate. The toluene was not substantially contained in the resulting concentrate, and the toluene content was almost 0%. From the toluene solution of 7-fluoro-1,1,3-trimethyl-4-aminoindane, the toluene was removed in a similar manner, to obtain 7-fluoro-1,1,3-trimethyl-4-aminoindane. The toluene was not substantially contained in the resulting concentrate, and the toluene content was almost 0%.

Subsequently, using the thus obtained ethyl 3-(difluoromethyl)-1-methyl-1H-pyrazole-4-carboxylate and 7-fluoro -1,1,3-trimethyl-4-aminoindane, synthesis of 3-difluoromethyl-N-(7-fluoro-1,1,3-trimethyl-4-indanyl)-1-methyl-4-pyrazole carboxamide was performed in the following manner.

To 43.0 mL of N-methylpyrrolidone in a 200-mL four-necked flask equipped with a cooling water refluxing tube, 20.2 g (98.9 mmol) of ethyl 3-(difluoromethyl)-1-methyl-1H-pyrazole-4-carboxylate and 17.4 g (90.0 mmol) of 7-fluoro-1,1,3-trimethyl-4-aminoindane were added.

The contents were stirred to form a solution, to which was then added 13.5 g (198.2 mmol) of sodium ethoxide, followed by stirring at an internal temperature of 48.9°C for 5.5 hours. The reaction mixture was cooled to 45°C, and then, 44.7 g of water was added to wash the organic layer. After separating the aqueous layer, 73.7 g of water was added to the organic layer, and cooling to 10°C was performed, whereby the target 3-difluoromethyl-N-(7-fluoro-1,1,3-trimethyl-4-indanyl)-1-methyl-4-pyrazole carboxamide was deposited as a crystal. The deposited crystal was separated by means of filtration and washed with 15.8 g of water.

The crystal after washing was dried. As a result, 30.6 g (83.8 mmol) of 3-difluoromethyl-N-(7-fluoro-1,1,3-trimethyl-4-indanyl)-1-methyl-4-pyrazole carboxamide was obtained as a white crystal. The yield was 93.0%, and the purity was 96.2%, and a purification operation, such as recrystallization, was not needed. Melting point: 170.8°C
¹H-NMR (1H resonance frequency: 500 MHz, measurement solvent: CDCl₃, internal standard substance: tetramethylsilane)

δ value (ppm): 1.43 (3H,d), 1.38 (3H,s), 1.44 (3H,s), 1.66 (1H,dd), 2.21 (1H,dd), 3.38 (1H,m), 3.93 (3H,s), 6.81 (1s,bs), 6.95 (1H,t), 6.70 (1H,m), 7.81 (1H,bs), 8.03 (1H,bs)

### Synthesis Example 1-4

Using the ethyl 3-(difluoromethyl)-1-methyl-1H-pyrazole-4-carboxylate and 7-fluoro-1,1,3-trimethyl-4-aminoindane as prepared in Synthesis Example 1-3, synthesis of 3-difluoromethyl- N-(7-fluoro-1,1,3-trimethyl-4-indanyl)-1-methyl-4-pyrazole carboxamide was performed in the following manner.

To 14.1 mL of N,N-dimethylacetamide in a 100-mL four-necked flask equipped with a cooling water refluxing tube, 6.86 g (33.3 mmol) of ethyl 3-(difluoromethyl)-1-methyl-1H-pyrazole-4-carboxylate and 5.80 g (30.0 mmol) of 7-fluoro-1,1,3-trimethyl-4-aminoindane were added.

The contents were stirred to form a solution, to which was then added 4.6 g (67.6 mmol) of sodium ethoxide, followed by stirring at an internal temperature of 85°C for 1.0 hour.

To the resulting reaction mixture, 32.0 g of water was added, and cooling to 8°C was performed, whereby the target 3-difluoromethyl-N-(7-fluoro-1,1,3-trimethyl-4-indanyl)-1-methyl-4-pyrazole carboxamide was deposited as a crystal. The deposited crystal was separated by means of filtration and washed with 25.0 g of water.

The crystal after washing was dried. As a result, 9.9 g (27.4 mmol) of 3-difluoromethyl-N-(7-fluoro-1,1,3-trimethyl-4-indanyl)-1-methyl-4-pyrazole carboxamide was obtained as a white crystal. The yield was 91.4%, and the purity was 97.3%, and a purification operation, such as recrystallization, was not needed. Melting point: 170.8°C
¹H-NMR (1H resonance frequency: 500 MHz, measurement solvent: CDCl₃, internal standard substance: tetramethylsilane)

δ value (ppm): 1.43 (3H,d), 1.38 (3H,s), 1.44 (3H,s), 1.66 (1H,dd), 2.21 (1H,dd), 3.38 (1H,m), 3.93 (3H,s), 6.81 (1s,bs), 6.95 (1H,t), 6.70 (1H,m), 7.81 (1H,bs), 8.03 (1H,bs)

### Synthesis Example 1-5

Using the ethyl 3-(difluoromethyl)-1-methyl-1H-pyrazole-4-carboxylate and 7-fluoro-1,1,3-trimethyl-4-aminoindane as prepared in Synthesis Example 1-3, synthesis of 3-difluoromethyl-N-(7-fluoro-1,1,3-trimethyl-4-indanyl)-1-methyl-4-pyrazole carboxamide was performed in the following manner.

To 10.7 mL of dimethyl sulfoxide in a 100-mL four-necked flask equipped with a cooling water refluxing tube, 6.7 g (32.8 mmol) of ethyl 3-(difluoromethyl)-1-methyl-1H-pyrazole-4-carboxylate and 5.8 g (30.0 mmol) of 7-fluoro-1,1,3-trimethyl-4-aminoindane were added.

The contents were stirred to form a solution, to which was then added 4.6 g (67.6 mmol) of sodium ethoxide, followed by stirring at an internal temperature of 84°C for 1.0 hour.

To the resulting reaction mixture, 32.8 g of water was added, and cooling to 10°C was performed, whereby the target 3-difluoromethyl-N-(7-fluoro-1,1,3-trimethyl-4-indanyl)-1-methyl-4-pyrazole carboxamide was deposited as a crystal. The deposited crystal was separated by means of filtration and washed with 50.0 g of water.

The crystal after washing was dried. As a result, 10.3 g (28.2 mmol) of 3-difluoromethyl-N-(7-fluoro-1,1,3-trimethyl-4-indanyl)-1-methyl-4-pyrazole carboxamide was obtained as a white crystal. The yield was 94.1%, and the purity was 96.3%, and a purification operation, such as recrystallization, was not needed. Melting point: 170.8°C
¹H-NMR (1H resonance frequency: 500 MHz, measurement solvent: CDCl₃, internal standard substance: tetramethylsilane)

δ value (ppm): 1.43 (3H,d), 1.38 (3H,s), 1.44 (3H,s), 1.66 (1H,dd), 2.21 (1H,dd), 3.38 (1H,m), 3.93 (3H,s), 6.81 (1s,bs), 6.95 (1H,t), 6.70 (1H,m), 7.81 (1H,bs), 8.03 (1H,bs)

### Reference Example 1-1

As an example of the conventional method, an example of performing the synthesis according to the method described in PTL 10 (WO 2012/055864 A) is shown as Reference Example 1-1.

Preparation of 3-difluoromethyl-N-(7-fluoro-1,1,3-trimethyl-4-indanyl)-1-methyl-4-pyrazole carboxamide

To 48.8 mL of tetrahydrofuran in a 100-mL four-necked flask equipped with a cooling water refluxing tube, 6.2 g (30.3 mmol) of ethyl 3-(difluoromethyl)-1-methyl-1H-pyrazole-4-carboxylate and 5.8 g (30.0 mmol) of 7-fluoro-1,1,3-trimethyl-4-aminoindane were added.

The contents were stirred to form a solution, and subsequently, a suspension liquid having added 5.2 g (45.0 mmol) of tert-butoxypotassium and 73.0 mL of tetrahydrofuran added in a 200-mL four-necked flask equipped with a cooling water refluxing tube was dropped, followed by stirring at an internal temperature of 25°C for 5.0 hours.

To the reaction mixture, 50.5 g of water and 156.8 g of ethyl acetate were added, and the organic layer was extracted twice. The extracted organic layer was washed with 29.4 g of water and 40.0 g of a saturated saline solution, and then, the organic layer was concentrated.

The residue was purified by means of silica gel chromatography (SiO₂; hexane/ethyl acetate = 1/1), to obtain 7.82 g (21.0 mmol) of the target 3-difluoromethyl-N-(7-fluoro-1,1,3-trimethyl-4-indanyl)-1-methyl-4-pyrazole carboxamide as a white crystal. The yield was 70.0%, and the purity was 94.4%. Melting point: 170.3°C
¹H-NMR (1H resonance frequency: 500 MHz, measurement solvent: CDCl₃, internal standard substance: tetramethylsilane)

δ value (ppm): 1.43 (3H,d), 1.38 (3H,s), 1.44 (3H,s), 1.66 (1H,dd), 2.21 (1H,dd), 3.38 (1H,m), 3.93 (3H,s), 6.81 (1s,bs), 6.95 (1H,t), 6.70 (1H,m), 7.81 (1H,bs), 8.03 (1H,bs)

In the light of the above, it is noted that in the synthesis method according to the present invention, on the occasion of the reaction, it is not needed to remove the by-produced alcohol or phenol outside the reaction system, to allow the reaction to proceed while shifting the equilibrium toward the reaction production system as in Reference Example 1-1, and thus, the reaction can be completed without removing the by-produced alcohol or phenol outside the reaction system; and moreover, on the occasion of the treatment, such as isolation, after the reaction, an operation, such as extraction, is not needed, and the pyrazole-4-carboxamide derivative can be obtained in high yield and high purity by an extremely simple method in which after completion of the reaction, water is added, cooling is performed to deposit a crystal, and the crystal is subjected to solid-liquid separation.

### Example 2

### Synthesis of 3-(difluoromethyl)-N-phenyl-1-methyl-4-pyrazole carboxamide (Compound No. 2)

To 48.1 mL of N-methylpyrrolidone in a 300-mL four-necked flask, 22.46 g (0.110 mol) of ethyl 3-(difluoromethyl)-1-methyl-1H-pyrazole-4-carboxylate and 9.42 g (0.100 mol) of aniline were added in a nitrogen atmosphere.

Subsequently, 13.61 g (0.20 mol) of sodium ethoxide was added, and the contents were stirred at an internal temperature of 81°C for 3 hours. The reaction mixture was cooled to 68°C, and then, 99.55 g of water was added, and cooling to 1.5°C was performed, whereby a crystal was deposited. The crystal was separated by means of filtration and washed with 99.62 g of water. The crystal after washing was dried. As a result, 24.26 g of 3-(difluoromethyl)-1-methyl-N-phenyl-4-pyrazole carboxamide was obtained as a white crystal. The yield was 95.5% (on a basis of aniline), and the purity was 99.9% (HPLC area percentage), and a purification operation, such as recrystallization, was not needed.
Melting point: 108.7°C
¹H-NMR (1H resonance frequency: 500 MHz, measurement solvent: CDCl₃, internal standard substance: tetramethylsilane)

δ value (ppm): 3.92 (3H,s), 6.92 (1H,s), 7.14 (1H,t), 7.35 (2H,t), 7.59 (2H,d), 8.00 (1H,s), 8.17 (1H,s)

### Example 3

### Synthesis of 3-(difluoromethyl)-N-(3'-methylphenyl)-1-methyl-4-pyrazole carboxamide (Compound No. 3)

To 50.3 mL of N-methylpyrrolidone in a 300-mL four-necked flask, 23.36 g (0.114 mol) of ethyl 3-(difluoromethyl)-1-methyl-1H-pyrazole-4-carboxylate and 11.13 g (0.104 mol) of 3-methylaniline were added in a nitrogen atmosphere.

Subsequently, 14.14 g (0.208 mol) of sodium ethoxide was added, and the contents were stirred at an internal temperature of 80°C for 2 hours. The reaction mixture was cooled to 69°C, and then, 103.58 g of water was added, and cooling to 2°C was performed, whereby a crystal was deposited. The crystal was separated by means of filtration and washed with 77.97 g of water. The crystal after washing was dried. As a result, 25.57 g of 3-(difluoromethyl)-N-(3'-methylphenyl)-1-methyl-4-pyrazole carboxamide was obtained as a white crystal. The yield was 92.8% (on a basis of 3-methylaniline), and the purity was 99.7% (HPLC area percentage), and a purification operation, such as recrystallization, was not needed.
Melting point: 110.8°C
¹H-NMR (1H resonance frequency: 500 MHz, measurement solvent: CDCl₃, internal standard substance: tetramethylsilane)

δ value (ppm): 2.36 (3H,s), 3.94 (3H,s), 6.90 (1H,t), 6.96 (1H,d), 7.24 (1H,t), 7.90 (1H,d), 7.44 (1H,s), 8.00 (1H,s), 8.08 (1H,bs)

### Example 4

### Synthesis of 3-(difluoromethyl)-1-methyl-N-(3'-fluorophenyl)-4-pyrazole carboxamide (Compound No. 4)

To 48.2 mL of N-methylpyrrolidone in a 300-mL four-necked flask, 22.45 g (0.110 mol) of ethyl 3-(difluoromethyl)-1-methyl-1H-pyrazole-4-carboxylate and 11.35 g (0.102 mol) of 3-fluoroaniline were added in a nitrogen atmosphere.

Subsequently, 13.70 g (0.201 mol) of sodium ethoxide was added, and the contents were stirred at an internal temperature of 80°C for 2.5 hours. The reaction mixture was cooled to 67°C, and then, 100.29 g of water was added, and cooling to 2°C was performed, whereby a crystal was deposited. The crystal was separated by means of filtration and washed with 53.56 g of water. The crystal after washing was dried. As a result, 26.16 g of 3-(difluoromethyl)-1-methyl-N-(3'-fluorophenyl)-4-pyrazole carboxamide was obtained as a white crystal. The yield was 95.1% (on a basis of 3-fluoroaniline), and the purity was 99.9% (HPLC area percentage), and a purification operation, such as recrystallization, was not needed.
Melting point: 120.7°C
¹H-NMR (1H resonance frequency: 500 MHz, measurement solvent: DMSOd₆, internal standard substance: tetramethylsilane)

δ value (ppm): 3.96 (3H,s), 6.91 (1H,dt), 7.31 (1H,t), 7.34-7.43 (2H,m), 7.66 (1H,td), 8.48 (1H,s), 10.19 (1H,bs)

### Example 5

### Synthesis of 3-(difluoromethyl)-1-methyl-N-(2',6'-dimethylphenyl)-4-pyrazole carboxamide (Compound No. 5)

To 48.3 mL of N-methylpyrrolidone in a 300-mL four-necked flask, 22.47 g (0.110 mol) of ethyl 3-(difluoromethyl)-1-methyl-1H-pyrazole-4-carboxylate and 13.77 g (0.102 mol) of 2,6-dimethylaniline were added in a nitrogen atmosphere.

Subsequently, 13.66 g (0.201 mol) of sodium ethoxide was added, and the contents were stirred at an internal temperature of 80°C for 2 hours. The reaction mixture was cooled to 67°C, and then, 100.79 g of water was added, and cooling to 2°C was performed, whereby a crystal was deposited. The crystal was separated by means of filtration and washed with 59.60 g of water. The crystal after washing was dried. As a result, 18.32 g of 3-(difluoromethyl)-1-methyl-N-(2',6'-dimethylphenyl)-4-pyrazole carboxamide was obtained as a white crystal. The yield was 64.1% (on a basis of 2,6-dimethylaniline), and the purity was 99.7% (HPLC area percentage), and a purification operation, such as recrystallization, was not needed.
Melting point: 158.6°C
¹H-NMR (1H resonance frequency: 500 MHz, measurement solvent: CDCl₃, internal standard substance: tetramethylsilane)

δ value (ppm): 2.27 (6H,s), 3.90 (3H,s), 6.90 (1H,t), 7.10-7.16 (3H,m), 7.74 (1H,bs), 7.97 (1H,s)

### Example 6

### Synthesis of 3-(difluoromethyl)-1-methyl-N-(2-biphenyl)-4-pyrazole carboxamide (Compound No. 6)

To 38.6 mL of N-methylpyrrolidone in a 300-mL four-necked flask, 17.97 g (0.088 mol) of ethyl 3-(difluoromethyl)-1-methyl-1H-pyrazole-4-carboxylate and 13.78 g (0.081 mol) of 2-biphenylamine were added in a nitrogen atmosphere.

Subsequently, 10.97 g (0.161 mol) of sodium ethoxide was added, and the contents were stirred at an internal temperature of 80°C for 2 hours. The reaction mixture was cooled to 69°C, and then, 78.81 g of water was added, and cooling to 2°C was performed, whereby a crystal was deposited. The crystal was separated by means of filtration and washed with 40.25 g of water. The crystal after washing was dried. As a result, 23.10 g of 3-(difluoromethyl)-1-methyl-N-(2-biphenyl)-4-pyrazole carboxamide was obtained as a white crystal. The yield was 86.7% (on a basis of 2-biphenylamine), and the purity was 96.7% (HPLC area percentage), and a purification operation, such as recrystallization, was not needed.
Melting point: 127.4°C
¹H-NMR (1H resonance frequency: 500 MHz, measurement solvent: CDCl₃, internal standard substance: tetramethylsilane)

δ value (ppm): 3.88 (3H,s), 6.75 (1H,t), 7.20-7.29 (2H,m), 7.37-7.47 (6H,m), 7.70 (1H,s), 7.80 (1H,bs), 8.29 (1H,d)

### Example 7

### Synthesis of 3-(difluoromethyl)-1-methyl-N-(3',4'-dichloro-5-fluorobiphenyl-2-yl)-4-pyrazole carboxamide (Compound No. 7: a general name: bixafen)

To 38.8 mL of N-methylpyrrolidone in a 300-mL four-necked flask, 18.8 g (0.0921 mol) of ethyl 3-(difluoromethyl)-1-methyl-1H-pyrazole-4-carboxylate and 21.72 g (0.085 mol) of 3',4'-dichloro-5-fluorobiphenyl-2-ylamine were added in a nitrogen atmosphere.

The 3',4'-dichloro-5-fluorobiphenyl-2-ylamine was prepared by reference to WO 2006/024388 A.

Subsequently, 11.56 g (0.170 mol) of sodium ethoxide was added, and the contents were stirred at an internal temperature of 80°C for 2 hours. The reaction mixture was cooled to 69°C, and then, 78.71 g of water was added, and cooling to 2°C was performed, whereby a crystal was deposited. The crystal was separated by means of filtration and washed with 40.30 g of water. The crystal after washing was dried. As a result, 33.10 g of 3-(difluoromethyl)-1-methyl-N-(3', 4'-dichloro-5-fluorobiphenyl-2-yl)-4-pyrazole carboxamide was obtained as a white crystal. The yield was 94.0% (on a basis of 3',4'-dichloro-5-fluorobiphenyl-2-ylamine), and the purity was 98.8% (HPLC area percentage), and a purification operation, such as recrystallization, was not needed.
Melting point: 146.1°C
¹H-NMR (1H resonance frequency: 500 MHz, measurement solvent: CDCl₃, internal standard substance: tetramethylsilane)

δ value (ppm): 3.91 (3H,s), 6.67 (1H,t), 6.972 (1H,dd), 7.10-7.14 (1H,m), 7.20 (1H,dd), 7.47-7.51 (2H,m), 7.73 (1H,bs), 7.90 (1H,s), 8.09 (1H,dd)

### Example 8

### Synthesis of 3-(difluoromethyl)-1-methyl-N-(3',4',5'-trifluorobiphenyl-2-yl)-4-pyrazole carboxamide (Compound No. 8: a general name: fluxapyroxad)

To 38.6 mL of N-methylpyrrolidone in a 300-mL four-necked flask, 17.97 g (0.088 mol) of ethyl 3-(difluoromethyl)-1-methyl-1H-pyrazole-4-carboxylate and 13.78 g (0.081 mol) of 3',4',5'-trifluorobiphenyl-2-ylamine were added in a nitrogen atmosphere.

The 3',4',5'-trifluorobiphenyl-2-ylamine was prepared by reference to WO 2010/094736 A.

Subsequently, 10.97 g (0.161 mol) of sodium ethoxide was added, and the contents were stirred at an internal temperature of 80°C for 2 hours. The reaction mixture was cooled to 69°C, and then, 78.90 g of water was added, and cooling to 2°C was performed, whereby a crystal was deposited. The crystal was separated by means of filtration and washed with 40.30 g of water. The crystal after washing was dried. As a result, 28.52 g of 3-(difluoromethyl)-1-methyl-N-(3',4',5'-trifluorobiphenyl-2-yl)-4-pyrazole carboxamide was obtained as a white crystal. The yield was 92.3% (on a basis of 3',4',5'-trifluorobiphenyl-2-ylamine), and the purity was 97.7% (HPLC area percentage), and a purification operation, such as recrystallization, was not needed.
Melting point: 156.2°C
¹H-NMR (1H resonance frequency: 500 MHz, measurement solvent: CDCl₃, internal standard substance: tetramethylsilane)

δ value (ppm): 3.92 (3H,s), 6.64 (1H,t), 6.99-7.03 (2H,m), 7.20-7.25 (2H,m), 7.41-7.45 (1H,m), 7.81 (1H,bs), 7.96 (1H,s), 8.14 (1H,d)

### Example 9

### Synthesis of 2-syn isomer: 3-(difluoromethyl)-1-methyl-N-[(1RS,4SR,9RS)-1,2,3,4-tetrahydro-9-isopropyl-1,4-methanonaphthalen-5-yl]pyrazole-4-caboxamide and 2-anti isomer: 3-(difluoromethyl)-1-methyl-N-[(1RS,4SR,9SR)-1,2,3,4-tetrahydro-9-isopropyl-1,4-methanonaphthalen-5-yl]pyrazole-4-caboxamide (Compound No. 9 obtained as a mixture of the syn-form and the anti-form : a general name: isopyrazam)

To 48.1 mL of N-methylpyrrolidone in a 300-mL four-necked flask, 22.50 g (0.110 mol) of ethyl 3-(difluoromethyl)-1-methyl-1H-pyrazole-4-carboxylate and 20.13 g (0.100 mol) of a mixture of N-[(1RS,4SR,9RS)-1,2,3,4-tetrahydro-9-isopropyl-1,4-methanonaphthalen-5-yl]a mine and N-[(1RS,4SR,9SR)-1,2,3,4-tetrahydro-9-isopropyl-1,4-methanonaphthalen-5-yllamine were added in a nitrogen atmosphere.

The mixture of N-[(1RS,4SR,9RS)-1,2,3,4-tetrahydro-9-isopropyl-1,4-methanonaphthalen-5-yl]amine and N-[(1RS,4SR,9SR)-1,2,3,4-tetrahydro-9-isopropyl-1,4-methanonaphthalen-5-yl]a mine was prepared by reference to WO 2007/115765 A (PTL 4).

Subsequently, 13.61 g (0.200 mol) of sodium ethoxide was added, and the contents were stirred at an internal temperature of 81°C for 3 hours. The reaction mixture was cooled to 68°C, and then, 99.80 g of water was added, and cooling to 1.5°C was performed, whereby a crystal was deposited. The crystal was separated by means of filtration and washed with 100.00 g of water. The crystal after washing was dried. As a result, 33.07 g of a mixture of a 2-syn isomer: 3-(difluoromethyl)-1-methyl-N-[(1RS,4SR,9RS)-1,2,3,4-tetrahydro-9-isopropyl-1,4-methanonaphthalen-5-yl]pyrazole-4-caboxamide and a 2-anti isomer: 3- (difluoromethyl)-1-methyl-N- [(1RS, 4SR,9SR)-1, 2, 3, 4-tetrahydro-9-isopropyl-1, 4-methanonaphthalen-5-yl]pyrazole-4-caboxamide was obtained as a white crystal. The yield was 92.0% (on a basis of the mixture of N-[(1RS,4SR,9RS)-1,2,3,4-tetrahydro-9-isopropyl-1,4-methanonaphthalen-5-yl]amine and N-[(1RS,4SR,9SR)-1,2,3,4-tetrahydro-9-isopropyl-1,4-methanonaphthalen-5-yl] amine), and the purity was 99.5% (the total sum of HPLC area percentages of the syn-form and the anti-form), and a purification operation, such as recrystallization, was not needed.
Melting point: 130.0°C (syn-form) and 144.4°C (anti-form)
¹H-NMR (1H resonance frequency: 500 MHz, measurement solvent: CDCl₃, internal standard substance: tetramethylsilane)

δ value (ppm): syn-form: 0.8 (6H,s), 1.0 (1H,m), 1.2-2.0 (5H,m), 3.2-3.4 (2H,m), 4.0 (3H,s), 6.6-8.2 (6H,m), and anti-form: 0.9 (6H,s), 1.0-2.0 (6H,m), 3.2-3.4 (2H,m), 4.0 (3H,s), 6.7-8.1 (6H,m)

### Example 10

### Synthesis of 3-(difluoromethyl)-1-methyl-N-[(1RS,4SR)-9-(dichloromethylene)-1,2,3,4-tetrahydro-1,4-methanonaphthalen-5-yl]pyrazole-4-carboxamide (Compound No. 10: a general name: benzovindiflupyr)

To 13.0 mL of N-methylpyrrolidone in a 300-mL four-necked flask, 6.09 g (0.030 mol) of ethyl 3-(difluoromethyl)-1-methyl-1H-pyrazole-4-carboxylate and 6.62 g (0.027 mol) of N-[(1RS,4SR)-9-(dichloromethylene)-1,2,3,4-tetrahydro-1,4-methanonaphthalen-5-yllamine were added in a nitrogen atmosphere.

The N-[(1RS,4SR)-9-(dichloromethylene)-1,2,3,4-tetrahydro-1,4-methanonaphthalen-5-yl]amine was prepared by reference to WO 2010/049228 A.

Subsequently, 3.70 g (0.054 mol) of sodium ethoxide was added at an internal temperature of 7.0°C, the temperature was raised to an internal temperature of 65°C over 2 hours, and the contents were stirred at the same temperature for 8 hours. The reaction mixture was cooled to 63°C, and then, 26.87 g of water was added, and cooling to 4.4°C was performed, whereby a crystal was deposited. The crystal was separated by means of filtration and washed with 152.60 g of water. The crystal after washing was dried. As a result, 10.31 g of 3-(difluoromethyl)-1-methyl-N-[(1RS,4SR)-9-(dichloromethylene)-1,2,3,4-tetrahyd ro-1,4-methanonaphthalen-5-yl]pyrazole-4-carboxamide was obtained as a white crystal. The yield was 95.8% (on a basis of N-[(1RS,4SR)-9-(dichloromethylene)-1,2,3,4-tetrahydro-1,4-methanonaphthalen-5 -yl]amine), and the purity was 98.4% (HPLC area percentage), and a purification operation, such as recrystallization, was not needed.
Melting point: 147.4°C
¹H-NMR (1H resonance frequency: 500 MHz, measurement solvent: CDCl₃, internal standard substance: tetramethylsilane)

δ value (ppm): 1.38 (1H,m), 1.49 (1H,m), 2.10 (2H,m), 3.95 (3H,s, overlapped by 1H,m), 4.06 (1H,m), 6.89 (1H,t, J = 54Hz), 7.03 (1H,d), 7.17 (1H,t), 7.82 (1H,d), 8.06 (1H,s), 8.11 (1H,bs)

### Example 11

### Synthesis of (RS)-N-[2-(1,3-dimethylbutyl)-3-thienyl]-1-methyl-3-(trifluoromethyl)-1H-pyrazole-4-carboxamide (Compound No. 11: a general name: penthiopyrad)

To 17.9 mL of N-methylpyrrolidone in a 300-mL four-necked flask, 24.56 g (0.111 mol) of ethyl 3-(trifluoromethyl)-1-methyl-1H-pyrazole-4-carboxylate and 18.43 g (0.101 mol) of (RS)-N-[2-(1,3-dimethylbutyl)-3-thienyl]amine were added in a nitrogen atmosphere.

The (RS)-N-[2-(1,3-dimethylbutyl)-3-thienyl]amine was prepared by reference to EP 0737682 A (PTL 6).

Subsequently, 13.65 g (0.201 mol) of sodium ethoxide was added, and the contents were stirred at an internal temperature of 70°C for 7 hours. To the reaction mixture, 48.30 g of water was added at the same temperature, and after stirring for 30 minutes, the resultant was allowed to stand at 65°C, to separate the aqueous layer. The organic layer was naturally cooled to 12.0°C over 12 hours while stirring. Subsequently, 49.58 g of water was added to the organic layer, followed by stirring at 30°C for 30 minutes. As a result, a crystal was gradually deposited. After stirring at the same temperature for 4 hours, the crystal was separated by means of filtration and washed with 77.60 g of water. The crystal after washing was dried. As a result, 33.44 g of (RS)-N-[2-(1,3-dimethylbutyl)-3-thienyl]-1-methyl-3-(trifluoromethyl)-1H-pyrazole-4-carboxamide was obtained as a white crystal. The yield was 93.2% (on a basis of (RS)-N-[2-(1,3-dimethylbutyl)-3-thienyl]amine), and the purity was 99.8% (HPLC area percentage), and a purification operation, such as recrystallization, was not needed.
Melting point: 108.5°C
¹H-NMR (1H resonance frequency: 500 MHz, measurement solvent: CDCl₃, internal standard substance: tetramethylsilane)

δ value (ppm): 0.86 (6H,d), 1.25 (3H,d), 1.4-1.7 (3H,m), 3.09 (1H,m), 3.94 (3H,s), 7.11 (1H,d), 7.39 (1H,s), 7.61 (1H,bs), 8.02 (1H,s)

In the light of the above, different from the present invention, the background art is in general concerned with the usual aminolysis reaction in which a by-produced alcohol or phenol is removed outside the reaction system through fractionation or the like, and the equilibrium is shifted toward the reaction production system, thereby completing the reaction, and it may be said that the treatment after the reaction is also usual one. On the other hand, the present invention is concerned with a technology in which the pyrazole-4-caroxamide derivative can be obtained in high yield and high purity by a simple method in which the reaction can be completed without removing the by-produced alcohol or phenol outside the system, and as for the treatment after the reaction, water is added after the end of the reaction, to deposit a crystal, and thus, it is noted that the present invention is concerned with an extremely simple and safe method.

### Industrial Applicability

The production method of the present invention is useful as an industrial production method in which the pyrazole-4-carboxamide derivative that is useful as an agricultural fungicide can be obtained in high yield and high purity through a short process and a simple operation.

## Claims

1. A method of producing a pyrazole-4-carboxamide derivative represented by the formula (1): 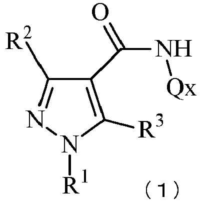 wherein,
R¹ is a hydrogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group,
R² is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group,
R³ is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, and
Qx is selected from Q1, Q2, Q3, Q4, Q5, and Q6: 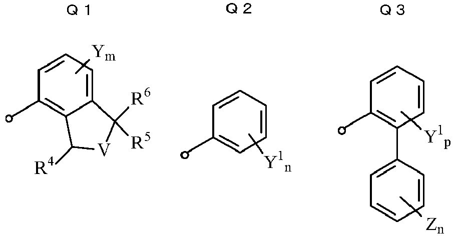 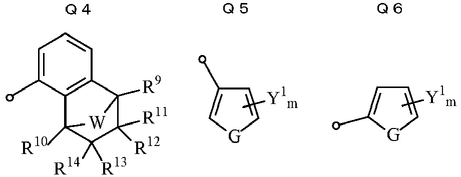
in Q1, R⁴, R⁵, and R⁶ are the same as or different from each other and are each a C1-C4 alkyl group, a C1-C4 haloalkyl group, a C3-C6 cycloalkyl group, or a C3-C6 halocycloalkyl group, provided that R⁵ and R⁶ may be bonded to each other to form a C3-C6 cycloalkyl group,
V represents CH(R⁷), N(R⁸), an oxygen atom, or a sulfur atom, and R⁷ and R⁸ are each a hydrogen atom, a C1-C4 alkyl group, a C1-C4 haloalkyl group, a C3-C6 cycloalkyl group, or a C3-C6 halocycloalkyl group,
Y represents a halogen atom, a C1-C4 alkyl group, a C1-C4 haloalkyl group, a C1-C4 alkoxy group, a C1-C4 haloalkoxy group, an SH group, a C1-C4 alkylthio group, or a C1-C4 haloalkylthio group, and
m is an integer of 0 to 3;
in Q2, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and
n is an integer of 1 to 5, and when n is 2, 3, 4, or 5, then Y¹'s may be the same as or different from each other;
in Q3, Y¹ and Z each represent a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group,
p represents an integer of 1 to 4, and when p is 2, 3, or 4, then Y¹'s may be the same as or different from each other, and
n represents an integer of 1 to 5, and when n is 2, 3, 4, or 5, then Z's may be the same as or different from each other;
in Q4, R⁹, R¹⁰, R¹¹, R¹², R¹³, and R¹⁴ are the same as or different from each other and each represent a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, R¹¹ and R¹² may be bonded to each other to form a C3-C6 cycloalkyl group and R¹³ and R¹⁴ may be bonded to each other to form a C3-C6 cycloalkyl group, and
W represents a methylene group, a methine group substituted with a C1-C6 haloalkyl group, or a terminal end-substituted vinyl group represented by the formula (4): 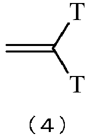
wherein T represents a C1-C6 haloalkyl group or a halogen atom;
in Q5, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group,
m represents an integer of 1 to 3, and when m is 2 or 3, then Y¹'s may be the same as or different from each other, and
G represents an oxygen atom, a sulfur atom, or N(R¹⁵), and R¹⁵ represents a hydrogen atom or a C1-C6 alkyl group; and
in Q6, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group,
m represents an integer of 1 to 3, and when m is 2 or 3, then Y¹'s may be the same as or different from each other, and
G represents an oxygen atom, a sulfur atom, or N(R¹⁵), and R¹⁵ represents a hydrogen atom or a C1-C6 alkyl group,
the method comprising subjecting a pyrazole-4-carboxylic acid ester represented by the formula (2): 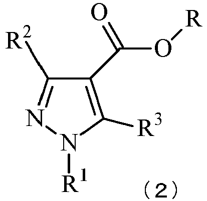
wherein,
R¹ is a hydrogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group,
R² is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group,
R³ is a hydrogen atom, a halogen atom, a C1-C4 alkyl group, a C1-C4 haloalkyl group, a C3-C6 cycloalkyl group, a C3-C6 halocycloalkyl group, a C1-C4 alkoxy group, a C1-C4 haloalkoxy group, a C1-C4 alkylthio group, a C1-C4 haloalkylthio group, an aralkyl group, or an aryl group, and
R is a C1-C4 alkyl group or an optionally substituted phenyl group,
and an amine represented by the formula (3):
H₂N-Qx (3)
wherein
Qx represents any substituent (amine residue) of Q1, Q2, Q3, Q4, Q5, and Q6: 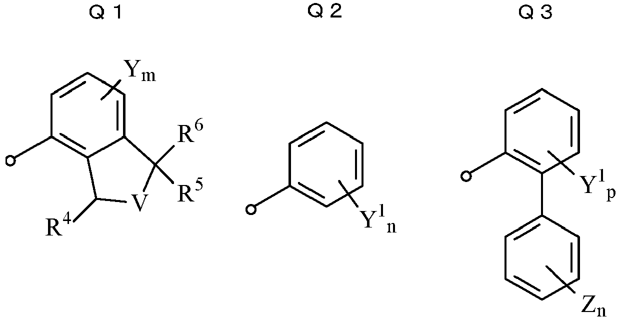 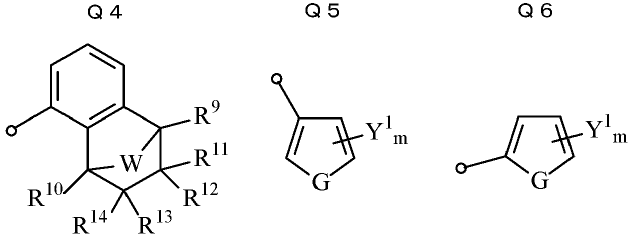
in Q1, R⁴, R⁵, and R⁶ are the same as or different from each other and are each a C1-C4 alkyl group, a C1-C4 haloalkyl group, a C3-C6 cycloalkyl group, or a C3-C6 halocycloalkyl group, provided that R⁵ and R⁶ may be bonded to each other to form a C3-C6 cycloalkyl group,
V represents CH(R⁷), N(R⁸), an oxygen atom, or a sulfur atom, and R⁷ and R⁸ are each a hydrogen atom, a C1-C4 alkyl group, a C1-C4 haloalkyl group, a C3-C6 cycloalkyl group, or a C3-C6 halocycloalkyl group,
Y represents a halogen atom, a C1-C4 alkyl group, a C1-C4 haloalkyl group, a C1-C4 alkoxy group, a C1-C4 haloalkoxy group, an SH group, a C1-C4 alkylthio group, or a C1-C4 haloalkylthio group, and
m is an integer of 0 to 3;
in Q2, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, and
n is an integer of 1 to 5, and when n is 2, 3, 4, or 5, then Y's may be the same as or different from each other;
in Q3, Y¹ and Z each represent a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group,
p represents an integer of 1 to 4, and when p is 2, 3, or 4, then Y's may be the same as or different from each other, and
n represents an integer of 1 to 5, and when n is 2, 3, 4, or 5, then Z's may be the same as or different from each other;
in Q4, R⁹, R¹⁰, R¹¹, R¹², R¹³, and R¹⁴ are the same as or different from each other and each represent a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group, R¹¹ and R¹² may be bonded to each other to form a C3-C6 cycloalkyl group, and R¹³ and R¹⁴ may be bonded to each other to form a C3-C6 cycloalkyl group, and
W represents a methylene group, a methine group substituted with a C1-C6 haloalkyl group, or a terminal end-substituted vinyl group represented by the formula (4): 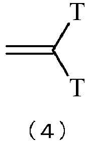
wherein T represents a C1-C6 haloalkyl group or a halogen atom;
in Q5, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group,
m represents an integer of 1 to 3, and when m is 2 or 3, then Y¹'s may be the same as or different from each other, and
G represents an oxygen atom, a sulfur atom, or N(R¹⁵), and R¹⁵ represents a hydrogen atom or a C1-C6 alkyl group; and
in Q6, Y¹ represents a hydrogen atom, a halogen atom, a C1-C6 alkyl group, a C1-C6 haloalkyl group, a C1-C6 alkoxy group, a C1-C6 haloalkoxy group, an SH group, a C1-C6 alkylthio group, or a C1-C6 haloalkylthio group,
m represents an integer of 1 to 3, and when m is 2 or 3, then Y¹'s may be the same as or different from each other, and
G represents an oxygen atom, a sulfur atom, or N(R¹⁵), and R¹⁵ represents a hydrogen atom or a C1-C6 alkyl group,
to an aminolysis reaction in a solvent in the presence of a base, provided that the reaction is completed without removing a by-produced alcohol or phenol,
wherein the solvent to be used for the reaction is an aprotic solvent,
the method further comprising:
(i) after completion of the reaction, adding water to the reaction mixture to deposit the target material, and subjecting the resultant to filtration, followed by drying, to thereby obtain a pyrazole-4-carboxamide derivative, or
(ii) after completion of the reaction, adding water to the reaction mixture, separating the aqueous layer to remove an unnecessary material from the organic layer containing the target material, adding water to the remaining organic layer to deposit the target material, and subjecting the resultant to filtration, followed by drying, to thereby obtain a pyrazole-4-carboxamide derivative.

2. The production method according to claim 1, wherein the base to be used for the reaction is a metal alkoxide.

3. The production method according to claim 2, wherein the metal alkoxide is at least one selected from lithium methoxide, lithium ethoxide, sodium methoxide, sodium ethoxide, potassium methoxide, and potassium ethoxide.

4. The production method according to any one of claims 1 to 3, wherein, as for a charging ratio of the pyrazole carboxylic acid ester represented by the formula (2) and the amine represented by the formula (3), the amount of the pyrazole carboxylic acid ester is in a range of 1.0 to 2.0 equivalents to 1.0 equivalent of the amine.

5. The production method according to any one of claims 1 to 4, wherein the use amount of the base is in a range of 1.0 to 7.0 equivalents to 1.0 equivalent of the amine represented by the formula (3).

6. The production method according to any one of claims 1 to 5, wherein the solvent to be used for the reaction is selected from the group consisting of N,N-dimethylacetamide, N-methylpyrrolidone, N,N-dimethylformamide and dimethyl sulfoxide.

7. The production method according to any one of claims 1 to 6, wherein the use amount of the solvent is in a range of 0.5 to 10.0 equivalents relative to the amine represented by the formula (3).

8. The production method according to any one of claims 1 to 7, wherein the amount of water needed for deposition in the above (i) and (ii) is about 1.0 to 7.0 in terms of a mass ratio relative to the used solvent.

## Patentansprüche

1. Verfahren zur Herstellung eines Pyrazol-4-carboxamid-Derivats, dargestellt durch die Formel (1): 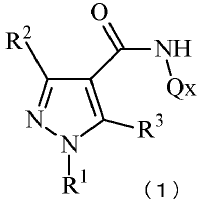 worin,
R¹ ein Wasserstoffatom, eine C1-C4-Alkylgruppe, eine C1-C4-Halogenalkylgruppe, eine C3-C6-Cycloalkylgruppe, eine C3-C6-Halogencycloalkylgruppe, eine C1-C4-Alkoxygruppe, eine C1-C4-Halogenalkoxygruppe, eine C1-C4-Alkylthiogruppe, eine C1-C4-Halogenalkylthiogruppe, eine Aralkylgruppe oder eine Arylgruppe ist,
R² ein Wasserstoffatom, ein Halogenatom, eine C1-C4-Alkylgruppe, eine C1-C4-Halogenalkylgruppe, eine C3-C6-Cycloalkylgruppe, eine C3-C6-Halogencycloalkylgruppe, eine C1-C4-Alkoxygruppe, eine C1-C4-Halogenalkoxygruppe, eine C1-C4-Alkylthiogruppe, eine C1-C4-Halogenalkylthiogruppe, eine Aralkylgruppe oder eine Arylgruppe ist,
R³ ein Wasserstoffatom, ein Halogenatom, eine C1-C4-Alkylgruppe, eine C1-C4-Halogenalkylgruppe, eine C3-C6-Cycloalkylgruppe, eine C3-C6-Halogencycloalkylgruppe, eine C1-C4-Alkoxygruppe, eine C1-C4-Halogenalkoxygruppe, eine C1-C4-Alkylthiogruppe, eine C1-C4-Halogenalkylthiogruppe, eine Aralkylgruppe oder eine Arylgruppe ist, und
Qx ausgewählt ist aus Q1, Q2, Q3, Q4, Q5 und Q6: 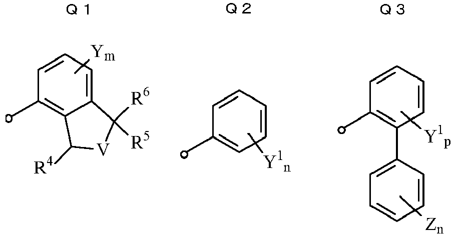 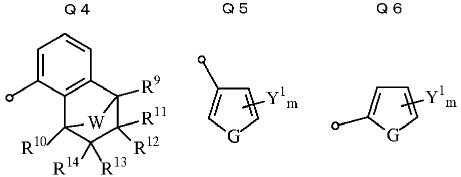
in Q1 sind R⁴, R⁵, und R⁶ gleich oder verschieden voneinander und sind jeweils eine C1-C4-Alkylgruppe, eine C1-C4-Halogenalkylgruppe, eine C3-C6-Cycloalkylgruppe oder eine C3-C6-Halogencycloalkylgruppe, mit der Maßgabe, dass R5 und R6 aneinander gebunden sein können, um eine C3-C6-Cycloalkylgruppe zu bilden,
V CH(R⁷), N(R⁸), ein Sauerstoffatom oder ein Schwefelatom darstellt und R⁷ und R⁸ jeweils ein Wasserstoffatom, eine C1-C4-Alkylgruppe, eine C1-C4-Halogenalkylgruppe, eine C3-C6-Cycloalkylgruppe oder eine C3-C6-Halogencycloalkylgruppe sind,
Y ein Halogenatom, eine C1-C4-Alkylgruppe, eine C1-C4-Halogenalkylgruppe, eine C1-C4-Alkoxygruppe, eine C1-C4-Halogenalkoxygruppe, eine SH-Gruppe, eine C1-C4-Alkylthiogruppe oder eine C1-C4-Halogenalkylthiogruppe darstellt, und
m eine ganze Zahl von 0 bis 3 ist;
in Q2 stellt Y¹ ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe dar, und
n ist eine ganze Zahl von 1 bis 5, und wenn n 2, 3, 4 oder 5 ist, dann können die Y¹'s gleich oder verschieden voneinander sein;
in Q3 stellen Y¹ und Z jeweils ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe dar,
p eine ganze Zahl von 1 bis 4 darstellt, und wenn p 2, 3 oder 4 ist, dann können die Y¹'s gleich oder verschieden voneinander sein, und
n eine ganze Zahl von 1 bis 5 darstellt, und wenn n 2, 3, 4 oder 5 ist, dann können die Z' s gleich oder verschieden voneinander sein;
in Q4 sind R⁹, R¹⁰, R¹¹, R¹², R¹³ und R¹⁴ gleich oder verschieden voneinander und stellen jeweils ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe darstellen, R¹¹ and R¹² aneinander gebunden sein können, um eine C3-C6-Cycloalkylgruppe zu bilden, und R¹³ und R¹⁴ aneinander gebunden sein können, um eine C3-C6-Cycloalkylgruppe zu bilden, und
W eine Methylengruppe, eine mit einer C1-C6-Halogenalkylgruppe substituierte Methingruppe oder eine endständig substituierte Vinylgruppe, dargestellt durch die Formel (4), darstellt: 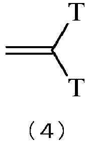
worin T eine C1-C6-Halogenalkylgruppe oder ein Halogenatom darstellt;
in Q5, Y¹ ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe darstellt,
m eine ganze Zahl von 1 bis 3 darstellt, und wenn m 2 oder 3 ist, dann können die Y¹'s gleich oder verschieden voneinander sein, und
G ein Sauerstoffatom, ein Schwefelatom oder N(R¹⁵) darstellt, und R¹⁵ ein Wasserstoffatom oder eine C1-C6-Alkylgruppe darstellt; und
in Q6 bedeutet Y¹ ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe,
m eine ganze Zahl von 1 bis 3 darstellt, und wenn m 2 oder 3 ist, dann können die Y¹'s gleich oder verschieden voneinander sein, und
G ein Sauerstoffatom, ein Schwefelatom oder N(R¹⁵), darstellt, und R¹⁵ ein Wasserstoffatom oder eine C1-C6-Alkylgruppe darstellt,
wobei das Verfahren das Unterwerfen eines Pyrazol-4-carbonsäureesters, der durch die Formel (2) dargestellt wird, wobei 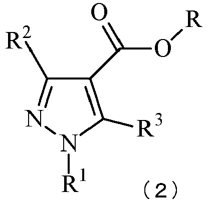 worin,
R¹ ein Wasserstoffatom, eine C1-C4-Alkylgruppe, eine C1-C4-Halogenalkylgruppe, eine C3-C6-Cycloalkylgruppe, eine C3-C6-Halogencycloalkylgruppe, eine C1-C4-Alkoxygruppe, eine C1-C4-Halogenalkoxygruppe, eine C1-C4-Alkylthiogruppe, eine C1-C4-Halogenalkylthiogruppe, eine Aralkylgruppe oder eine Arylgruppe ist,
R² ein Wasserstoffatom, ein Halogenatom, eine C1-C4-Alkylgruppe, eine C1-C4-Halogenalkylgruppe, eine C3-C6-Cycloalkylgruppe, eine C3-C6-Halogencycloalkylgruppe, eine C1-C4-Alkoxygruppe, eine C1-C4-Halogenalkoxygruppe, eine C1-C4-Alkylthiogruppe, eine C1-C4-Halogenalkylthiogruppe, eine Aralkylgruppe Gruppe oder eine Arylgruppe,
R³ ist ein Wasserstoffatom, ein Halogenatom, eine C1-C4-Alkylgruppe, eine C1-C4-Halogenalkylgruppe, eine C3-C6-Cycloalkylgruppe, eine C3-C6-Halogencycloalkylgruppe, eine C1-C4-Alkoxygruppe, eine C 1 -C4-Halogenalkoxygruppe, eine C1-C4-Alkylthiogruppe, eine C1-C4-Halogenalkylthiogruppe, eine Aralkylgruppe oder eine Arylgruppe, und
Reine C1-C4-Alkylgruppe oder eine gegebenenfalls substituierte Phenylgruppe ist, und ein Amin, dargestellt durch die Formel (3):
H₂N-Qx ( 3)
worin
Qx einen beliebigen Substituenten (Aminrest) von Q1, Q2, Q3, Q4, Q5 und Q6 darstellt: 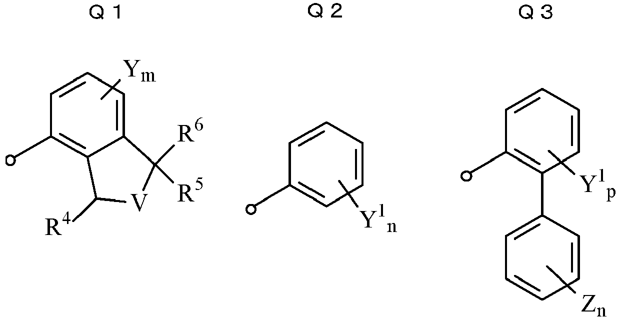 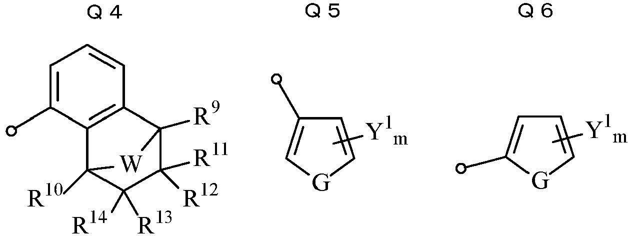 in Q1 sind R⁴, R⁵ und R⁶ gleich oder verschieden voneinander und sind jeweils eine C1-C4-Alkylgruppe, eine C1-C4-Halogenalkylgruppe, eine C3-C6-Cycloalkylgruppe oder eine C3-C6-Halogencycloalkylgruppe, mit der Maßgabe, dass R5 und R6 aneinander gebunden sein können, um eine C3-C6-Cycloalkylgruppe zu bilden,
V CH(R⁷), N(R⁸), ein Sauerstoffatom oder ein Schwefelatom darstellt und R⁷ und R⁸ jeweils ein Wasserstoffatom, eine C1-C4-Alkylgruppe, eine C1-C4-Halogenalkylgruppe, eine C3-C6-Cycloalkylgruppe oder eine C3-C6-Halogencycloalkylgruppe sind,
Y ein Halogenatom, eine C1-C4-Alkylgruppe, eine C1-C4-Halogenalkylgruppe, eine C1-C4-Alkoxygruppe, eine C1-C4-Halogenalkoxygruppe, eine SH-Gruppe, eine C1-C4-Alkylthiogruppe oder eine C1-C4-Halogenalkylthiogruppe ist, und
m eine ganze Zahl von 0 bis 3 ist;
in Q2 stellt Y¹ ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe dar, und
n eine ganze Zahl von 1 bis 5 ist, und wenn n 2, 3, 4 oder 5 ist, dann können die Y's gleich oder verschieden voneinander sein;
in Q3 bedeuten Y¹ und Z jeweils ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe,
p eine ganze Zahl von 1 bis 4 darstellt, und wenn p 2, 3 oder 4 ist, dann können die Y's gleich oder verschieden voneinander sein, und
n eine ganze Zahl von 1 bis 5 darstellt, und wenn n 2, 3, 4 oder 5 ist, dann können die Z' s gleich oder verschieden voneinander sein;
in Q4 sind R⁹, R¹⁰, R¹¹, R¹², R¹³ und R¹⁴ gleich oder verschieden voneinander und stellen jeweils ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe darstellen, R¹¹und R¹² aneinander gebunden sein können, um eine C3-C6-Cycloalkylgruppe zu bilden, und R¹³ und R¹⁴ aneinander gebunden sein können, um eine C3-C6-Cycloalkylgruppe zu bilden, und
W eine Methylengruppe, eine mit einer C1-C6-Halogenalkylgruppe substituierte Methingruppe oder eine endständig substituierte Vinylgruppe, dargestellt durch die Formel (4), darstellt: 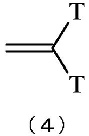
worin T eine C1-C6-Halogenalkylgruppe oder ein Halogenatom darstellt;
in Q5, Y¹ ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe darstellt,
m eine ganze Zahl von 1 bis 3 darstellt, und wenn m 2 oder 3 ist, dann können die Y¹'s gleich oder verschieden voneinander sein, und
G ein Sauerstoffatom, ein Schwefelatom oder N(R¹⁵) darstellt, und R¹⁵ für eine Wasserstoffatom oder eine C1-C6-Alkylgruppe; und
in Q6 bedeutet Y¹ ein Wasserstoffatom, ein Halogenatom, eine C1-C6-Alkylgruppe, eine C1-C6-Halogenalkylgruppe, eine C1-C6-Alkoxygruppe, eine C1-C6-Halogenalkoxygruppe, eine SH-Gruppe, eine C1-C6-Alkylthiogruppe oder eine C1-C6-Halogenalkylthiogruppe,
m eine ganze Zahl von 1 bis 3 darstellt, und wenn m 2 oder 3 ist, dann können die Y¹'s gleich oder verschieden voneinander sein, und
G ein Sauerstoffatom, ein Schwefelatom oder Ndarstellt(R¹⁵), und R¹⁵ein Wasserstoffatom oder eine C1-C6-Alkylgruppe darstellt,
einer Aminolyse-Reaktion in einem Lösungsmittel in Gegenwart einer Base, mit der Maßgabe, dass die Reaktion ohne Entfernung eines als Nebenprodukt gebildeten Alkohols oder Phenols abgeschlossen wird,
wobei das für die Reaktion zu verwendende Lösungsmittel ein aprotisches Lösungsmittel ist,
wobei das Verfahren ferner umfasst:
(i) nach Beendigung der Reaktion Zugabe von Wasser zu der Reaktionsmischung, um das Zielmaterial abzuscheiden, und Filtration des resultierenden Materials, gefolgt von Trocknung, um dadurch ein Pyrazol-4-carboxamid-Derivat zu erhalten, oder
(ii) nach Beendigung der Reaktion Zugabe von Wasser zu der Reaktionsmischung, Abtrennen der wässrigen Schicht, um ein unnötiges Material von der organischen Schicht, die das Zielmaterial enthält, zu entfernen, Zugabe von Wasser zu der verbleibenden organischen Schicht, um das Zielmaterial abzuscheiden, und Unterziehen des Resultats einer Filtration, gefolgt von Trocknen, um dadurch ein Pyrazol-4-carboxamid-Derivat zu erhalten.

2. Herstellungsverfahren nach Anspruch 1, wobei die für die Reaktion zu verwendende Base ein Metallalkoxid ist.

3. Herstellungsverfahren nach Anspruch 2, wobei das Metallalkoxid mindestens eines ist, ausgewählt aus Lithiummethoxid, Lithiumethoxid, Natriummethoxid, Natriumethoxid, Kaliummethoxid und Kaliumethoxid

4. Verfahren zur Herstellung nach einem der Ansprüche 1 bis 3, wobei die Menge des Pyrazolcarbonsäureesters der Formel (2) und des Amins der Formel (3) in einem Bereich von 1,0 bis 2,0 Äquivalenten zu 1,0 Äquivalenten des Amins liegt, was das Beladungsverhältnis betrifft.

5. Herstellungsverfahren nach einem der Ansprüche 1 bis 4, wobei die Verwendung Menge der Base in einem Bereich von 1,0 bis 7,0 Äquivalenten zu 1,0 Äquivalenten des durch die Formel (3) dargestellten Amins liegt.

6. Herstellungsverfahren nach einem der Ansprüche 1 bis 5, wobei das für die Reaktion zu verwendende Lösungsmittel ausgewählt ist aus der Gruppe bestehend aus N,N-Dimethylacetamid, N-Methylpyrrolidon, N,N-Dimethylformamid und Dimethylsulfoxid.

7. Herstellungsverfahren nach einem der Ansprüche 1 bis 6, wobei die eingesetzte Menge des Lösungsmittels in einem Bereich von 0,5 bis 10,0 Äquivalenten, bezogen auf das durch die Formel (3) dargestellte Amin, liegt.

8. Herstellungsverfahren nach einem der Ansprüche 1 bis 7, **dadurch gekennzeichnet, dass** die zur Abscheidung benötigte Wassermenge in den obigen (i) und (ii) etwa 1,0 bis 7,0 beträgt, bezogen auf das Massenverhältnis zum verwendeten Lösungsmittel.

## Revendications

1. Procédé de production d'un dérivé de pyrazole-4-carboxamide représenté par la formule (1) : 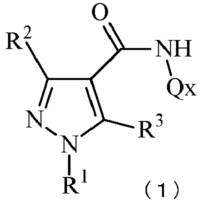 dans lequel,
R¹ est un atome d'hydrogène, un groupe alkyle en C1-C4, un groupe haloalkyle en C1-C4, un groupe cycloalkyle en C3-C6, un groupe halocycloalkyle en C3-C6, un groupe alcoxy en C1-C4, un groupe haloalcoxy en C1-C4, un groupe alkylthio en C1-C4, un groupe haloalkylthio en C1-C4, un groupe aralkyle ou un groupe aryle,
R² est un atome d'hydrogène, un atome d'halogène, un groupe alkyle en C1-C4, un groupe haloalkyle en C1-C4, un groupe cycloalkyle en C3-C6, un groupe halocycloalkyle en C3-C6, un groupe alcoxy en C1-C4, un groupe haloalcoxy en C1-C4, un groupe alkylthio en C1-C4, un groupe haloalkylthio en C1-C4, un groupe aralkyle ou un groupe aryle,
R³ est un atome d'hydrogène, un atome d'halogène, un groupe alkyle en C1-C4, un groupe haloalkyle en C1-C4, un groupe cycloalkyle en C3-C6, un groupe halocycloalkyle en C3-C6, un groupe alcoxy en C1-C4, un groupe haloalcoxy en C1-C4, un groupe alkylthio en C1-C4, un groupe haloalkylthio en C1-C4, un groupe aralkyle ou un groupe aryle, et
Qx est choisi parmi Q1, Q2, Q3, Q4, Q5 et Q6: 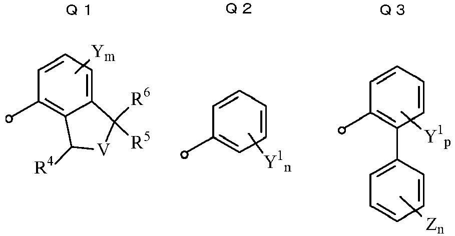 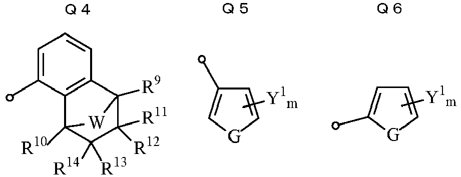
dans Q1, R⁴, R⁵ et R⁶ sont identiques ou différents les uns des autres et sont chacun un groupe alkyle en C1-C4, un groupe haloalkyle en C1-C4, un groupe cycloalkyle en C3-C6 ou un groupe halocycloalkyle en C3-C6, à condition que R⁵ et R⁶ puissent être liés l'un à l'autre pour former un groupe cycloalkyle en C3-C6,
V représente CH(R⁷), N(R⁸), un atome d'oxygène ou un atome de soufre, et R⁷ et R⁸ sont chacun un atome d'hydrogène, un groupe alkyle en C1-C4, un groupe haloalkyle en C1-C4, un groupe cycloalkyle en C3-C6 ou un groupe halocycloalkyle en C3-C6,
Y représente un atome d'halogène, un groupe alkyle en C1-C4, un groupe haloalkyle en C1-C4, un groupe alcoxy en C1-C4, un groupe haloalcoxy en C1-C4, un groupe SH, un groupe alkylthio en C1-C4 ou un groupe haloalkylthio en C1-C4, et
m est un nombre entier de 0 à 3;
dans Q2, Y¹ représente un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6, et
n est un nombre entier de 1 à 5, et lorsque n est 2, 3, 4 ou 5, les Y¹ peuvent être identiques ou différents les uns des autres ;
dans Q3, Y¹ et Z représentent chacun un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6,
p représente un nombre entier de 1 à 4, et lorsque p est 2, 3 ou 4, les Y¹ peuvent être identiques ou différents les uns des autres, et
n représente un nombre entier de 1 à 5, et lorsque n est 2, 3, 4 ou 5, les Z peuvent être identiques ou différents les uns des autres ;
dans Q4, R⁹, R¹⁰, R¹¹, R¹², R¹³ et R¹⁴ sont identiques ou différents les uns des autres et représentent chacun un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6, R¹¹ et R¹² peuvent être liés l'un à l'autre pour former un groupe cycloalkyle en C3-C6 et R¹³ et R¹⁴ peuvent être liés l'un à l'autre pour former un groupe cycloalkyle en C3-C6, et
W représente un groupe méthylène, un groupe méthine substitué par un groupe haloalkyle en C1-C6, ou un groupe vinyle substitué en bout de chaîne représenté par la formule (4): 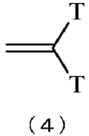
dans laquelle T représente un groupe haloalkyle en C1-C6 ou un atome d'halogène;
dans Q5, Y¹ représente un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6,
m représente un nombre entier de 1 à 3, et lorsque m est 2 ou 3, les Y¹ peuvent être identiques ou différents les uns des autres, et
G représente un atome d'oxygène, un atome de soufre ou N(R¹⁵), et R¹⁵ représente un atome d'hydrogène ou un groupe alkyle en C1-C6; et
dans Q6, Y¹ représente un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6,
m représente un nombre entier de 1 à 3, et lorsque m est 2 ou 3, les Y¹ peuvent être identiques ou différents les uns des autres, et
G représente un atome d'oxygène, un atome de soufre ou N(R¹⁵), et R¹⁵ représente un atome d'hydrogène ou un groupe alkyle en C1-C6,
le procédé comprenant la soumission d'un ester d'acide pyrazole-4-carboxylique représenté par la formule (2): 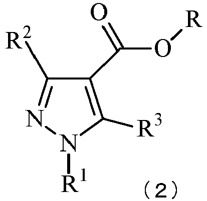
dans lequel,
R¹ est un atome d'hydrogène, un groupe alkyle en C1-C4, un groupe haloalkyle en C1-C4, un groupe cycloalkyle en C3-C6, un groupe halocycloalkyle en C3-C6, un groupe alcoxy en C1-C4, un groupe haloalcoxy en C1-C4, un groupe alkylthio en C1-C4, un groupe haloalkylthio en C1-C4, un groupe aralkyle ou un groupe aryle,
R² est un atome d'hydrogène, un atome d'halogène, un groupe alkyle en C1-C4, un groupe haloalkyle en C1-C4, un groupe cycloalkyle en C3-C6, un groupe halocycloalkyle en C3-C6, un groupe alcoxy en C1-C4, un groupe haloalcoxy en C1-C4, un groupe alkylthio en C1-C4, un groupe haloalkylthio en C1-C4, un groupe aralkyle ou un groupe aryle,
R³ est un atome d'hydrogène, un atome d'halogène, un groupe alkyle en C1-C4, un groupe haloalkyle en C1-C4, un groupe cycloalkyle en C3-C6, un groupe halocycloalkyle en C3-C6, un groupe alcoxy en C1-C4, un groupe haloalcoxy en C1-C4, un groupe alkylthio en C1-C4, un groupe haloalkylthio en C1-C4, un groupe aralkyle ou un groupe aryle, et
R est un groupe alkyle en C1-C4 ou un groupe phényle éventuellement substitué, et une amine représentée par la formule (3) :
H₂N-Qx (3)
où
Qx représente tout substituant (résidu d'amine) de Q1, Q2, Q3, Q4, Q5 et Q6 : 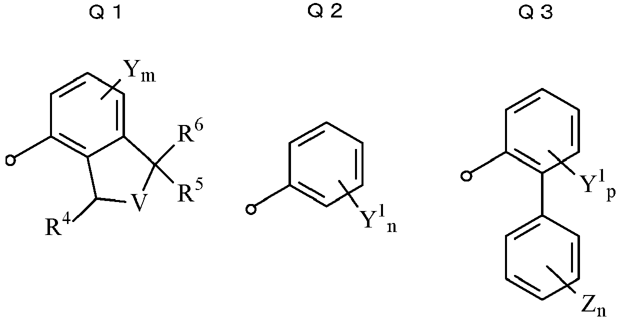 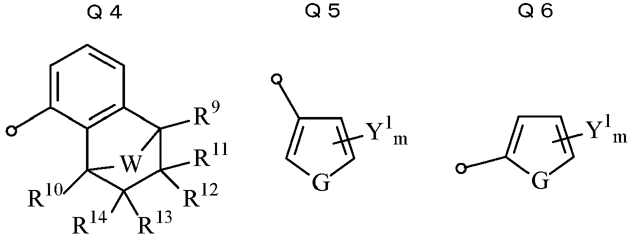
dans Q1, R⁴, R⁵ et R⁶ sont identiques ou différents les uns des autres et sont chacun un groupe alkyle en C1-C4, un groupe haloalkyle en C1-C4, un groupe cycloalkyle en C3-C6 ou un groupe halocycloalkyle en C3-C6, à condition que R⁵ et R⁶ puissent être liés l'un à l'autre pour former un groupe cycloalkyle en C3-C6,
V représente CH(R⁷ ), N(R⁸ ), un atome d'oxygène ou un atome de soufre, et R⁷ et R⁸ sont chacun un atome d'hydrogène, un groupe alkyle en C1-C4, un groupe haloalkyle en C1-C4, un groupe cycloalkyle en C3-C6 ou un groupe halocycloalkyle en C3-C6,
Y représente un atome d'halogène, un groupe alkyle en C1-C4, un groupe haloalkyle en C1-C4, un groupe alcoxy en C1-C4, un groupe haloalcoxy en C1-C4, un groupe SH, un groupe alkylthio en C1-C4 ou un groupe haloalkylthio en C1-C4, et
m est un nombre entier de 0 à 3;
dans Q2, Y¹ représente un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6, et
n est un nombre entier de 1 à 5, et lorsque n est 2, 3, 4 ou 5, les Y peuvent être identiques ou différents les uns des autres;
dans Q3, Y¹ et Z représentent chacun un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6,
p représente un nombre entier de 1 à 4, et lorsque p est 2, 3 ou 4, les Y peuvent être identiques ou différents les uns des autres, et
n représente un nombre entier de 1 à 5, et lorsque n est 2, 3, 4 ou 5, les Z peuvent être identiques ou différents les uns des autres ;
dans Q4, R⁹ , R¹⁰, R¹¹, R¹², R¹³ et R¹⁴ sont identiques ou différents les uns des autres et représentent chacun un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6, R¹¹ et R¹² peuvent être liés l'un à l'autre pour former un groupe cycloalkyle en C3-C6, et R¹³ et R¹⁴ peuvent être liés l'un à l'autre pour former un groupe cycloalkyle en C3-C6, et
W représente un groupe méthylène, un groupe méthine substitué par un groupe haloalkyle en C1-C6, ou un groupe vinyle substitué en bout de chaîne représenté par la formule (4) : 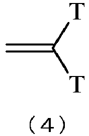
dans laquelle T représente un groupe haloalkyle en C1-C6 ou un atome d'halogène ;
dans Q5, Y¹ représente un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6,
m représente un nombre entier de 1 à 3, et lorsque m est 2 ou 3, les Y¹ peuvent être identiques ou différents les uns des autres, et
G représente un atome d'oxygène, un atome de soufre ou N(R¹⁵), et R¹⁵ représente un atome d'hydrogène ou un groupe alkyle en C1-C6; et
dans Q6, Y¹ représente un atome d'hydrogène, un atome d'halogène, un groupe alkyle en C1-C6, un groupe haloalkyle en C1-C6, un groupe alcoxy en C1-C6, un groupe haloalcoxy en C1-C6, un groupe SH, un groupe alkylthio en C1-C6, ou un groupe haloalkylthio en C1-C6,
m représente un nombre entier de 1 à 3, et lorsque m est 2 ou 3, les Y¹ peuvent être identiques ou différents les uns des autres, et
G représente un atome d'oxygène, un atome de soufre ou N(R¹⁵), et R¹⁵ représente un atome d'hydrogène ou un groupe alkyle en C1-C6,
à une réaction d'aminolyse dans un solvant en présence d'une base, à condition que la réaction soit achevée sans éliminer un alcool ou un phénol sous-produit,
dans lequel le solvant à utiliser pour la réaction est un solvant aprotique,
le procédé comprenant en outre:
(i) après achèvement de la réaction, ajouter de l'eau au mélange réactionnel pour déposer le matériau cible, et soumettre le produit résultant à une filtration, suivie d'un séchage, pour obtenir ainsi un dérivé de pyrazole-4-carboxamide, ou
(ii) après achèvement de la réaction, ajouter de l'eau au mélange réactionnel, séparer la couche aqueuse pour éliminer une matière non nécessaire de la couche organique contenant la matière cible, ajouter de l'eau à la couche organique restante pour déposer la matière cible, et soumettre le produit résultant à une filtration, suivie d'un séchage, pour obtenir ainsi un dérivé de pyrazole-4-carboxamide.

2. Procédé de production selon la revendication 1, dans lequel la base à utiliser pour la réaction est un alcoxyde métallique.

3. Procédé de production selon la revendication 2, dans lequel l'alcoxyde métallique est au moins un choisi parmi le méthoxyde de lithium, l'éthoxyde de lithium, le méthoxyde de sodium, l'éthoxyde de sodium, le méthoxyde de potassium et l'éthoxyde de potassium.

4. Procédé de production selon l'une quelconque des revendications 1 à 3, dans lequel, en ce qui concerne un rapport de charge de l'ester d'acide pyrazole carboxylique représenté par la formule (2) et de l'amine représentée par la formule (3), la quantité de l'ester d'acide pyrazole carboxylique est dans une gamme de 1,0 à 2,0 équivalents pour 1,0 équivalent de l'amine.

5. Procédé de production selon l'une quelconque des revendications 1 à 4, dans lequel la quantité d'utilisation de la base est dans une gamme de 1,0 à 7,0 équivalents pour 1,0 équivalent de l'amine représentée par la formule (3).

6. Procédé de production selon l'une quelconque des revendications 1 à 5, dans lequel le solvant à utiliser pour la réaction est choisi dans le groupe constitué par le N,N-diméthylacétamide, la N-méthylpyrrolidone, le N,N-diméthylformamide et le diméthylsulfoxyde.

7. Procédé de production selon l'une quelconque des revendications 1 à 6, dans lequel la quantité d'utilisation du solvant est dans une gamme de 0,5 à 10,0 équivalents par rapport à l'amine représentée par la formule (3).

8. Procédé de production selon l'une quelconque des revendications 1 à 7, dans lequel la quantité d'eau nécessaire au dépôt dans les (i) et (ii) ci-dessus est d'environ 1,0 à 7,0 en termes de rapport massique par rapport au solvant utilisé.
